# EUROPEAN PATENT SPECIFICATION

(11) **EP 1 809 749 B1**
(45) Date of publication and mention of the grant of the patent: **11.02.2009**
(21) Application number: 05810933.1
(22) Date of filing: 26.10.2005
(51) Int. Cl.: C12N 15/63, C12N 1/21

(54) **METHODS FOR HETEROLOGOUS EXPRESSION OF SECONDARY METABOLITES**
VERFAHREN ZUR HETEROLOGEN EXPRESSION SEKUNDÄRER METABOLITE
METHODES D'EXPRESSION HETEROLOGUE DE METABOLITES SECONDAIRES

(30) Priority: 26.10.2004 GB 0423755
(43) Date of publication of application: 25.07.2007
(73) Proprietor: Gene Bridges Gmbh, 01307 Dresden (DE)
(72) Inventor: ZHANG, Youming, 01309 Dresden (DE); MÜLLER, Rolf, 66440 Blieskastel (DE); STEWART, Francis, 01277 Dresden (DE); GROSS, Frank, 01099 Dresden (DE); WENZEL, Silke, C., 34292 Ahnatal (DE); FU, Jun, 01307 Dresden (DE)
(74) Representative: Goodfellow, Hugh Robin
(86) International application number: PCT/IB2005/003650
(87) International publication number: WO 2006/046152

(56) References cited:
- US-A1- 2004 005 672
- PFEIFER B A ET AL: "Biosynthesis of complex polyketides in a metabolically engineered strain of E. coli" SCIENCE, AMERICAN ASSOCIATION FOR THE ADVANCEMENT OF SCIENCE,, US, vol. 291, no. 5509, 2 March 2001 (2001-03-02), pages 1790-1792, XP002170306 ISSN: 0036-8075
- ZIRKLE ROSS ET AL: "Heterologous production of the antifungal polyketide antibiotic soraphen A of Sorangium cellulosum So ce26 in Streptomyces lividans" MICROBIOLOGY (READING), vol. 150, no. Part 8, August 2004 (2004-08), pages 2761-2774, XP002379636 ISSN: 1350-0872
- RODRIGUEZ E ET AL: "RAPID ENGINEERING OF POLYKETIDE OVERPRODUCTION BY GENE TRANSFER TO INDUSTRIALLY OPTIMIZED STRAINS" JOURNAL OF INDUSTRIAL MICROBIOLOGY AND BIOTECHNOLOGY, BASINGSTOKE, GB, vol. 30, no. 8, August 2003 (2003-08), pages 480-488, XP009027209 ISSN: 1367-5435
- WENZEL S C ET AL: "Heterologous Expression of a Myxobacterial Natural Products Assembly Line in Pseudomonads via Red/ET Recombineering" CHEMISTRY AND BIOLOGY, CURRENT BIOLOGY, LONDON, GB, vol. 12, no. 3, March 2005 (2005-03), pages 349-356, XP004818904 ISSN: 1074-5521
- WENZEL ET AL: "Recent developments towards the heterologous expression of complex bacterial natural product biosynthetic pathways" CURRENT OPINION IN BIOTECHNOLOGY, LONDON, GB, vol. 16, no. 6, December 2005 (2005-12), pages 594-606, XP005173491 ISSN: 0958-1669
- GROSS FRANK ET AL: "Bacterial type III polyketide synthases: phylogenetic analysis and potential for the production of novel secondary metabolites by heterologous expression in pseudomonads." ARCHIVES OF MICROBIOLOGY. MAR 2006, vol. 185, no. 1, March 2006 (2006-03), pages 28-38, XP002379637 ISSN: 0302-8933

## Description

The invention relates to methods for heterologous expression of secondary metabolites.

### Background

Many secondary metabolites, including commercially important antibiotics and cytotoxins, are produced in diverse prokaryotes and eukaryotes from enzymatic pathways encoded by gene complexes, which are often found in a large, single, contiguous genomic region. Because the structure of the secondary metabolite product of a biosynthetic pathway is directed by the specificity of the enzymes along the pathway, mutagenesis of the genes encoding the enzymes is potentially an advantageous way to alter the chemical product. Hence, variations in secondary metabolites, formerly limited to the applied science of organic chemistry, can be achieved through the application of DNA mutagenesis to the genes of these pathways.

Whereas organic chemistry is limited to the modification of high energy bond sites on the secondary metabolite, DNA mutagenesis can theoretically alter every bond in a secondary metabolite. Therefore DNA mutagenesis presents exceptional promise for the alteration of existing, and the creation of new, secondary metabolites for drug optimization and discovery. However, DNA mutagenesis technology, which is highly developed for *E.coli,* is poorly developed for the diverse hosts of relevance to secondary metabolite production. At best, current *in situ* host-by-host approaches for mutagenesis of secondary metabolite pathways are limited to individual mutagenesis that is often labour intensive.

In order to overcome the problems associated with the limited capacity of natural secondary metabolite producing hosts such as Streptomycetes for genetic manipulation, other heterologous hosts have been investigated. *E. coli* has been a preferred host cell as techniques for performing cloning and genetic manipulation in *E. coli* are well established in the art. For example Kealey et al., ('Production of a polyketide natural product in nonpolyketide-producing prokaryotic and eukaryotic hosts', PNAS USA, (1998), 95:505-509), describes the production of the fungal polyketide 6-methylsalicylic acid (6-MSA) in heterologous *E. coli,* yeast and plant cells. Further, Pfeifer el al., ('Biosynthesis of complex polyketides in a metabolically engineered strain of E. coli', Science (2001) 291: 1790-1792) describes the genetic engineering of a derivative of *E. coli* in which the resulting cellular catalyst converts exogenous propionate into the polyketide erythromycin (6-deoxyerythronolide B). The use of *E. coli* for engineering coupled with *Streptomyces* as the expression host has been described by scientists at the John Innes Institute in Norwich in Gust el al., ('PCR-targeted Streptomyces gene replacement identifies a protein domain needed for biosynthesis of the sesquiterpene soil odor geosmin.' PNAS USA (2003) 100:1541-1546).

However, the absence of certain precursor production pathways and enzymes required for biosynthesis limits the value of *E. coli* and the other heterologous host cells described in the art for heterologous expression of secondary metabolites. For example, *E. coli* lacks at least two activities required for most polyketide and non-ribosomal peptide (NRP) pathways. Whereas these activities can be introduced into *E. coli,* these engineered hosts produce only small amounts of the intended secondary metabolite. Furthermore, *E. coli* has a low GC genomic content, unlike the genomes of Actinomycetes and Myxobacteria, the major secondary metabolite producing hosts, which both have a high GC content. Thus, codon usage is not optimised in *E. coli* when a gene from these organisms is expressed.

There is a need for alternative and improved methods for heterologous expression which couple the advantages of fluent DNA mutagenesis and engineering whilst enabling good host properties for the production of secondary metabolites.

### Summary of the invention

Accordingly, in a first aspect, the invention provides a method for the heterologous production of a secondary metabolite encoded by a biosynthetic pathway, comprising:
i) generating in a first host cell, a single vector comprising the component genes of the biosynthetic pathway, wherein the vector is constructed using principles of recombineering;
ii) transforming a second host cell with the vector, wherein the second host cell is a Pseudomonas or Myxobacterium;
iii) culturing the second host cell under conditions which are suitable for synthesis of the secondary metabolite;
wherein the genes of the biosynthetic pathway are transcribed under the control of promoters that are found naturally in the second host cell.

Also provided is a method as recited in claim 2.

The methods of the invention allow, for the first time, the expression of complex metabolic pathways within host systems that are compatible with and able to support both the expression and activity of the proteins that form part of such pathways. These methods combine the simplicity of genetic manipulation in a first host cell, used for cloning purposes, with the properties of a second host cell that is more suitable for the expression and screening of secondary metabolites. This methodology allows the experience and technology acquired over many years of working with cloning hosts, such as *E. coli* and *Salmonella,* to be exploited, whilst utilising the much greater capacity of other, less well understood species as expression vehicles for generation of complex secondary metabolites.

The secondary metabolite expressed in the method of the invention may be known or unknown, but in most cases the invention will be utilised in the pursuit of novel, bioactive compounds. The secondary metabolite is preferably bioactive, and thus has useful biological properties. For example, the secondary metabolite may advantageously have antibiotic or cytotoxic properties.

Such compounds may be synthesised by a biosynthetic pathway encoded by a single gene or a biosynthetic pathway encoded by more than one gene. Preferably, the biosynthetic pathway is encoded by more than one gene. In such embodiments, the vector used in the methodology of the invention may comprise two, three, four, five or more of the genes of the biosynthetic pathway. If not all of the genes of the biosynthetic pathway are contained on the vector, the other genes required for activity of the pathway may be provided either on one or more additional vectors or may be integrated onto the chromosome of the second host cell, either naturally, or through directed chromosomal integration. Preferably, all the genes of the biosynthetic pathway that are not already present in the second host cell are encoded on a single vector.

Preferably, all of the genes of a particular biosynthetic pathway are encoded on a single vector. The establishment of a secondary metabolite pathway on a single DNA molecule is not only relevant for pathways that exist naturally as single contiguous clusters, but also for pathways that exist in more than one part, often in distinct regions of the genome. The parts can be cloned together into one molecule to simplify handling. For example, the presence of all the enzymes on one single vector enables the second host cell to be transformed so as to contain the pathway in one single step. In contrast, prior art methods, for example in which E *coli* has been modified to contain the genes for epothilone or erythromycin synthesis, have utilised several plasmids to transform the genes into *E. coli* and thus have required multiple transformation steps (Li et al., 'Cloning and Heterologous Expression of the Epothilone Gene Cluster' (2000) Science 287: 640-642). Further, in the methodology of the present invention, where the biosynthetic pathway is encoded by more than one gene, the presence of all the genes on a single vector enables the various enzymes of the biosynthetic pathway to be expressed at an equivalent stoichiometric ratio of 1:1. In this way, the expression of the genes is generally equivalent, governed by the principal of co-linearity, and is not influenced by the potentially different copy number of different vectors carrying different parts of the gene cluster.

Examples of suitable vectors will be known to those of skill in the art, and may be selected rationally to suit the requirements of any particular system, taking into account information known about the length of sequence to be cloned, the type of second host system to be used, and so on. Of particular suitability will be episomal and virus-derived systems derived from: bacterial plasmids, bacteriophage, cosmids and phagemids, and bacterial artificial chromosomes (BACs). BACs in particular may also be employed to deliver larger fragments of DNA than can be contained and expressed in a plasmid.

The component genes of the biosynthetic pathway may be comprised within a transposable element ("transposon") carried by the vector. The movement of transposable elements was described by Barbara McClintock in the 1940s and 1950s during her discovery of transposition in maize (Comfort NC. 2001. "From controlling elements to transposons: Barbara McClintock and the Nobel Prize", Trends Biochem. Sci. 26:454-57). Transposons are ubiquitous and they are present in nearly all organisms from prokaryotes to eukaryotes, including humans (Berg, D.E. and Howe, M.M., 1989, "Mobile DNA ", Washington, DC: ASM Press; Craig N.L. et al., 2002, "Mobile DNA II", Washington, DC: ASM Press; Merlin C, et al., 2000, «Gene recruiters and transporters: the modular structure of bacterial mobile elements", In The Horizontal Gene Pool, ed. CM Thomas, pp. 363-409. Amsterdam: Harwood Academic). The simplest transposon is a segment of DNA flanked by sequences (often these are inverted repeats) that are recognized by a protein - the transposase - which enables the transposon to transpose. The transposase randomly integrates the transposon into the chromosome.

Transposition technology is widely used nowadays. Its applications include *in vitro* transposition mutagenesis for DNA sequencing, *in vivo* insertional mutagenesis for functional gene studying and gene transfer. Gene transfer by using the Sleeping Beauty Transposon has been used in gene therapy (Ohlfest JR et al., "Combinatorial Antiangiogenic Gene Therapy by Nonviral Gene Transfer Using the Sleeping Beauty Transposon Causes Tumor Regression and Improves Survival in Mice Bearing Intracranial Human Glioblastoma", Mol Ther. 2005 Sep 5; [Epub ahead of print]) and its size limit has been studied in a mouse cell line (Karsi A. et al., 2001, "effects of insert size on transposition efficiency of the sleeping beauty transposon in mouse cells" Mar. Biotechnol. (NY)., 3(3):241-5). Large sized transposable elements (86kb) were successfully used for *in vitro* mutagenesis mediated by Tn5 transposase (Joydeep B. et al., 2005, "Rapid creation of BAC-based human artificial chromosome vectors by transposition with synthetic alpha-satellite arrays" Nucleic Acids Research. 33(2):587-596).

However, there have been no reports on the use of a transposon to introduce large sized DNA molecules (also described herein as large sized DNA "fragments") into a heterologous host. Transferring and integration of large sized DNA molecules into the chromosome in many bacterial strains is difficult because the efficiency of endogenous homologous recombination in these hosts is low. To overcome this, transposition technology serves as an alternative method for large size gene transfer.

Thus, in one embodiment, a transposable element may be used to introduce one or more large sized DNA molecules into the chromosome of a heterologous host. The large sized DNA molecule is introduced into the chromosome within a transposable element carried by a vector. The large sized DNA molecule is flanked by transposon sequences that are recognized by a transposase enzyme. Preferably, the large sized DNA molecule or molecules provide one or more component genes of a biosynthetic pathway for synthesising a secondary metabolite, as described herein. Preferably, the whole biosynthetic gene cluster is integrated into the transposable element. Preferably, the vector is a BAC. A second host cell may be transformed with the vector comprising large sized DNA molecule flanked by the transposon sequences recognised by the transposase using any suitable method, for example, by conjugation or electroporation.

Particularly preferred transposons for use in the invention belong to the *mariner* family of transposable elements. The *mariner* family of transposable elements is named for the original element discovered in *D. mauritiana* (Berg, D.E. and Howe, M.M., 1989, "Mobile DNA ", Washington, DC: ASM Press). They are small elements around 1,300 bp long with approximately 30 bp inverted terminal repeats, and they contain a single open reading frame encoding a transposase of about 345 amino acids (Robertson, H. M., 1993, "The mariner element is widespread in insects", Nature, 362:241-245; Robertson, H. M. 1995, "The Tel -mariner superfamily of transposons in animals", J. Insect Physiol., 41:99-105). The *mariner* family is most closely related to the Tcl family of transposons found in nematodes, *Drosophila,* and fish (Robertson, H. M. 1995, "The Tel -mariner superfamily of transposons in animals", J. Insect Physiol. 41:99-105; Henikoff, A. and Henikoff, J.G., 1992, "Amino acid substitution matrices from protein blocks", Proc. Natl. Acad Sci. USA. 89:10915-10919).

The most preferred transposon for transferring large sized DNA molecules is the MycoMar transposable element, which is a mariner transposon (Rubin, E. et al., 1999, "In vivo transposition of mariner-based elements in enteric bacteria and mycobacteria", Proc. Natl. Acad Sci. USA., 96:1645-1650).

By the term "large sized DNA molecule" is meant a DNA molecule of more than 20kb in length, for example 30-150 kb in length, 40-100kb in length, 50-80 kb in length, and so on. Large sized DNA molecules are preferably introduced into the transposon (for example, into the MycoMar transposable element) using recombineering (also known as "Red/ET recombination technology"), as described below. Red/ET recombination technology is an ideal tool for large size DNA engineering.

Where the component genes of the biosynthetic pathway are comprised within a transposable element carried by the vector, a suitable transposase is preferably also transformed into the second host cell. For example, where the MycoMar transposable element is used, the MycoMar transposase gene is also preferably transformed into the second host cell. Preferably, the transposase is under the control of a promoter that is not active in the first host, but is active in the second host. Expression of the transposase after the vector enters the second host cell integrates the transposable element into the chromosome. Preferably, the vector comprising the transposon also comprises the transposase gene. For example, the transposase gene is preferably cloned outside of the transposable element flanked by the inverted repeats. When the transposase gene itself is present in the non-replicatable vector backbone, its expression is lost after the initial phase of expression in the second host.

Engineering a gene cluster encoding the biosynthetic pathway for synthesising a secondary metabolite into a vector such as a transposable element, and introducing the engineered gene cluster into a heterologous host, opens a new window for drug development and production.

Preferably, according to the invention, the secondary metabolite is generated by a polyketide pathway, a non-ribosomal peptide (NRP) pathway or a fatty acid pathway or is synthesised by a pathway which combines enzymes from two or more of the pathways encoding these secondary metabolites, for example a hybrid potyketide-NRP.

Where the secondary metabolite is generated by a polyketide pathway, this pathway is preferably a type I polyketide pathway. However, the polyketide may be any other type of polyketide, for example a type II or a type III polyketide, such as flaviolin. An example of a secondary metabolite generated by a hybrid polyketide-NRP pathway is myxochromide. The myxochromide gene cluster is a preferred example of a biosynthetic pathway that can be exploited according to the present invention.

Preferably, the biosynthetic pathway is not endogenous to the second host cell. By this it is meant either that the pathway itself, in the form contained on the vector, is not naturally known in the second host cell, or that one or more of the genes that make up the pathway is not known in the second host cell.

Preferably, the secondary metabolite is not naturally produced in the second host cell. The method of the invention allows the study of pathways that are completely unknown in either of the host cell systems that are used.

According to the methodology of the invention; the genes of the biosynthetic pathway are transcribed under the control of promoters that are found naturally in the second host cell. This is an important element of the methodology of the invention, for it allows the transcription machinery of the second host cell to recognise its own promoters and thus transcribe the genes implicated in the metabolic pathway under study.

In previous work, this concept has not been expressed, workers instead relying on alternative mechanisms to effect expression of the pathway of interest, which have in general led to the production of a low level of the desired product. As briefly referred to above, such strategies have mainly relied on the manipulation of heterologous genes in *E. coli* hosts so as to use *E. coli-*derived promoters. One big disadvantage of this strategy is that *E. coli* is most unsuited to the expression of most biosynthetic pathways that are of interest in the context of the present invention. Another strategy is to use the host in which the gene cluster is naturally expressed, relying on the endogenous expression from naturally-used promoters in that host. However, the majority of hosts that naturally generate compounds of interest as bioactive compounds are either completely unstudied, or very little is known about them (for example, bacterial colonisers of sea sponges and the like), meaning that their culture and manipulation in the laboratory is not possible. This makes such a strategy limited to a very small selection of hosts, such as Streptococci.

The use of a first host cell in which genetic manipulation is simple allows the alteration of the promoters in the second cell, without undue difficulty. Standard tools may be used for this manipulation, including PCR. Preferably, however, recombineering methodologies are used to alter the promoters, as necessary (see International patent applications WO99/29837 and WO02/062988; European patent applications 01117529.6 and 0103276.2; United States patents 6,509,156 and 6,355,412; and also Muyrers, J.P.P. et al., 2000 ('ET-Cloning: Think Recombination First', Genetic Eng., vol. 22, 77-98), Muyrers, J.P et al., 2001 (Techniques: Recombinogenic engineering-new options for cloning and manipulating DNA, Trends in Biochem. Sci., 26, 325-31), Zhang, Y et al., 2000 (DNA cloning by homologous recombination in Escherichia coli., Nature Biotech., 18, 1314-1317), Muyrers J.P et al., 2000 (Point mutation of bacterial artificial chromosomes by ET recombination, EMBO Reports, 1, 239-243), Muyrers J.P et al., 2000 (RecE/RecT and Redαa/Redβa initiate double-stranded break repair by specifically interacting with their respective partners, Genes Dev., 14, 1971-1982), Muyrers et al., 1999 (Rapid modification of bacterial artificial chromosomes by ET-recombination, Nucleic Acid Res., 27, 1555-1557), Zhang Y. et al., 1998 (A new logic for DNA engineering using recombination in Escherichia coli, Nat. Genet., 20, 123-128) Narayanan K. et al., (Efficient and precise engineering of a 200 kb β-globin human/bacterial artificial chromosome in E. coli DH10B using an inducible homologous recombination system, Gene Therapy, 6, 442-447) and Zhang, Y. et al., 2003 (BMC Mol Biol. 2003 Jan 16; 4 (1):1)). Recombineering is a technique of great potential that has not yet found general application, partly because its potential has not been widely realised, and also because a degree of experience and expertise is required in order to exploit its potential fully.

One or more of the genes in the biosynthetic pathway may be cloned under the control of an inducible promoter. This will be particularly advantageous where the secondary metabolite is toxic to the second host cell, since it will mean that the pathway can be established in the host while quantities of the host are grown up unaffected by the potential toxicity of the secondary metabolite.

This novel approach is advantageous over those currently used in the art - existing systems that involve the expression of a toxic gene product generally circumvent the problem of toxicity by an alternative strategy, namely that of co-expressing a resistance gene that transports the toxic product out of the cell. In a system such as that described herein, the expression of a resistance gene is not feasible, as the nature of the (only potentially toxic) secondary metabolite being expressed is not known, for example, where the method is used to screen a library of secondary metabolites. Using an inducible promoter to govern expression of one or more of the genes necessary for production of the secondary metabolite allows the cells to grow to a high cell density before the inducing agent is added, and expression of a high level of the secondary metabolite is only induced at that point. If the metabolite is toxic, the cells will die, but while dying will still produce a sufficient quantity of secondary metabolite for further analysis or purification.

Preferred inducible promoters will be those which are induced by small molecules. Examples of suitable systems are known in the art, and include the toluic acid inducible Pm promoter in *Pseudomonas* species described by Abril M.A et al., 1989 (Regulator and enzyme specificities of the TOL plasmid-encoded upper pathway for degradation of aromatic hydrocarbons and expansion of the substrate range of the pathway, J. Bacteriol., 171:6782), which is an example of a preferred inducible promoter.

One advantage of the use of an inducible promoter, particularly in the context of a screen for bioactive metabolites (among the most interesting of which will be those with antibiotic or cytotoxic properties) is that host cell death upon promoter induction acts as a preliminary screen for those compounds that merit further investigation.

Thus, where a method of the present invention is used to express a secondary metabolite that is toxic to the second host cell, cell death may be used as an indication that the secondary metabolite is bioactive. The inventors have surprisingly found that even during the process of cell death due to the toxic secondary metabolite, the second host cell is still able to produce the secondary metabolite at a useful level which may be recoverable. Preferably, the inducible promoter will be one that can be regulated with small ligands so that potential toxic effects of the expressed secondary metabolite can be managed with ease.

Standard prior art DNA cloning methodologies can in principal produce DNA clones that are large enough to carry known secondary metabolite pathways. However, such cloning methodologies are random processes with no control maintained over the end points of the segment found in any one clone. As a result, large segments of randomly cloned DNA usually omit, at one end or the other, essential parts of the gene clusters in question, and/or include flanking sequences encoding irrelevant genes that could provoke undesired complications.

In contrast, the method of the present invention preferably exploits recombineering methodologies, which allow large stretches of DNA encoding the gene or genes encoding the enzymes of the biosynthetic pathway to be cloned effectively into one vector. Recombineering enables stretches of DNA of various sizes to be cloned into vectors, ranging from very short genes up to many kilobases, potentially encompassing gene clusters of more than 20 kb, for example 30-150 kb in length, 40-100kb in length, 50-80 kb in length, and so on, to be engineered and expressed in a system that allows their subsequent manipulation and analysis. For example, this allows, for the first time, the facility to clone a biosynthetic pathway as complex as the type I polyketides into a single vector. This was either not possible previously, or would require such extraordinary effort as to be impractical and thus unfeasible using cloning techniques currently used in the art, as these do not allow such large stretches of DNA to be engineered.

The method devised by the inventors involves the use of recombineering to clone the genes encoding the enzymes of the biosynthetic pathway, preferably onto a single vector. Thus, the vector is constructed in the first host cell using recombineering (see above). Recombineering is a method of cloning DNA which does not require *in vitro* treatments with restriction enzymes or DNA ligases and is therefore fundamentally distinct from the standard methodologies of DNA cloning. The method relies on a pathway of homologous recombination in *E. coli* involving the *recE* (endonuclease) and *recT* (phage annealing protein) gene products from the Rac prophage, or the *red*α and *red*β *gene* products from Lambda phage, and functionally equivalent gene products from other sources.

The use of the recombineering methodology carries with it the dual advantages of enabling both small and large DNA molecules to be engineered and also enabling other more subtle genetic manipulations, such as insertions, deletions and point mutations, to be performed in a restriction enzyme-independent fashion. This facility is of great significance when manipulating large stretches of nucleic acid, when restriction analysis becomes unfeasible. One such advantage of recombineering is the ability to allow promoters to be manipulated at will.

Another advantage comes from the realisation that in many cases, random starting clones will be engineered to create single clones that are optimized for the specific goal of screening for secondary metabolites of interest. Whereas conventional cloning methodologies require the sequence of the cloned material to be known, so that strategies can be designed to manipulate the genes of interest, recombineering does not require this and allows large stretches of nucleic acid of unknown sequence to be cloned and manipulated at will.

A further advantage of recombineering methods is that the recombined vector can be integrated into the genome of the second host cell, giving rise to transformed strains of this host with a stable insertion of the genes of desired biosynthetic pathway. This is an advantage over transformation of the second host with a plasmid or plasmids containing the genes of the biosynthetic pathway, which may, for example, be rearranged under transformation conditions or be lost from the transformed strain during culturing and storage.

The first host cell is preferably a host cell that allows the generation and maintenance of a vector for use in the method of the present invention. The first host cell is preferably a host cell for which genetic engineering techniques are well known in the art. Preferably, the first host cell is one in which recombineering methodologies may be effected, so as to allow manipulation of large stretches of nucleic acid of unknown sequence, as well as to perform more subtle, but equally necessary refinements such as promoter replacement.

The first host cell is also preferably a host cell that is able to conjugate efficiently with the second host cell. For example, *E. coli* is a preferred first host cell. However, other suitable first host cells include other gram negative bacteria, particularly those that are well studied such as *Pseudomonads* and *Salmonella* species. Methods for genetic engineering of *E. coli* and Salmonella are described in full in known laboratory manuals such as that by Sambrook et al., Molecular Cloning; A Laboratory Manual, Third Edition (2001).

The second host cell is a Pseudomonas or Myxobacterium.

Preferably, the second host cell is a cell which normally expresses secondary metabolites of the type in which there is an interest, particularly secondary metabolites of the class that is being expressed (i.e. generated by NRP pathways, type I polyketides pathways etc.). An appropriate choice of the second host cell will ensure that this host is well adapted for expression of the secondary metabolite. The second host cell may be a cell which does not naturally express the precise secondary metabolite of interest.

Certain host cells do not naturally express one or more of the substrates that are required for biosynthesis of certain classes of secondary metabolite. For example, type I polyketide synthases catalyze the successive condensation of carboxylic acid residues from their substrates such as malonyl-CoA and methylmalonyl-CoA. Malonyl-CoA is a substrate for primary metabolism pathway and is present in all bacteria. However, methylmalonyl-CoA (a second common precursor of polyketides) is not naturally produced in a wide range of bacterial strains.

A heterologous host for all kinds of polyketide gene cluster expression should synthesize methylmalonyl-CoA. Thus the second host cell is preferably transformed with genes encoding the enzymes required for making substrates that are required to synthesise the secondary metabolite but which are not naturally expressed in the wild-type second host cell. Preferably, the genes are integrated into the chromosome of the second host cell. Alternatively, a substrate which is not normally expressed in the second host cell may be induced to be expressed in the second host cell by replacement of the endogenous promoter governing expression of the appropriate gene with an appropriate constitutive or inducible promoter, and/or by culturing it under specific conditions.

Advantageously, the second host cell is a *Pseudomonas.* The inventors have established that the use of *Pseudomonas* as the host in which to synthesise the secondary metabolite is advantageous for a number of reasons. Principal among these is that, unlike most secondary metabolite producing hosts, *Pseudomonas* and Myxobacteria grow easily and rapidly in culture and their use is scalable for industrial production. Furthermore, *Pseudomonas* species are genetically more similar to Actinomycetes and Myxobacteria, the major secondary metabolite producing hosts, than hosts such as *E. coli,* that have proven ineffective for production of such compounds. For example, Actinomycetes and Myxobacteria both have a high GC genomic content, as does *Pseudomonas,* whereas *E. coli* has a low GC genomic content. This results in codon usage being more efficient in *Pseudomonas* than in *E. coli* since *Pseudomonas* has an endogenous codon usage profile that is very similar to that of both Myxobacteria and Actinomycetes. The codon usage profile of these species is very different to that of *E. coli.*

*Pseudomonas putida, P. stutzeri* and *P. syringae* are particularly preferred host cells for expression of the genes of the biosynthetic pathway. These host cells have been found to grow fast, facilitating culture on both a laboratory and industrial scale. Furthermore, *Pseudomonas putida* has, in particular, surprisingly been found to generate very high protein levels, when tested by the inventors. This clearly reinforces its suitability for use in the present invention, as the quantities of cell culture that need to be prepared are reduced by to as little as a third of what would be required using alternative systems.

As mentioned above, one problem that has frustrated those working this field so far is that certain host cells do not express one or more of the substrates that are required for biosynthesis of certain classes of secondary metabolite. *Pseudomonas,* on the other hand, can grow on valine as the sole carbon source. Under these conditions *Pseudomonas* may produce methylmalonyl CoA, one of the substrates required for polyketide synthesis. In the presence of other carbon sources methylmalonyl CoA expression could be induced by the replacement of the endogenous promoter governing expression of the appropriate gene with an appropriate constitutive or inducible promoter. It is preferable, according to the invention, to transform the *Pseudomonas* with the genes encoding the enzymes required to synthesise methylmalonyl CoA.

Another advantage that the inventors have identified in using *Pseudomonas* as a second host cell in the context of the invention is that this bacterium is capable of producing heterologous secondary metabolites from particular complex gene clusters that require the activity of phosphopantetheinyl (Ppant) -dependent carrier proteins. These must be made functionally active by transfer of the 4'-Ppant moiety from coenzyme A in order for polyketide synthases and non-ribosomal peptide synthases to function. This step is catalyzed by an enzyme called P-pant transferase. For example, the apo form of polyketide synthase enzymes is synthesized from their gene clusters and is converted to the holo form by adding a phosphopantetheinyl (P-pant) moiety to a serine residue of the acyl or peptidyl carrier protein (ACP or PCP) domains. P-pant transferase is not produced in a wide range of bacterial strains and so it may be necessary to transform the second host that is used in a method of the invention with a gene encoding a P-pant transferase. Generally, in previous work, dedicated host Ppant transferases have been used to catalyse reactions of this type. However, the inventors have discovered that *P. putida, P. stuizeri* and *P. syringae* naturally contain a broad specificity Ppant transferase that effectively activates peptidyl carrier proteins (PCPs) and acyl carrier proteins (ACPs) {see example 3]. Thus, these *Pseudomonas* species are able to activate heterologous PCPs and ACPs with CoA using endogenous Ppant transferase activity. This quality makes *Pseudomonas* particularly suitable second hosts for the expression of polyketide biosynthetic pathways. Thus when a Pseudomonas is used as the second host cell, it is not necessary to transform the Pseudomonas with an enzyme encoding a P-pant transferase, unless the presence of an exogenous P-pant transferase is desired.

Further, in contrast to most secondary metabolite producing hosts, *Pseudomonas* can be readily transformed with DNA using physical methods such as calcium phosphate transformation and electroporation. It also has excellent endogenous properties for homologous recombination, which enables efficient integration into the endogenous genome for stable maintenance of introduced DNA molecules.

The use of a combination of *E. coli* as the first host cell and *Pseudomonas,* particularly *P. putida,* as the second host cell is a particularly preferred combination for use in the present invention. *Pseudomonas* is known to conjugate efficiently with *E. coli,* so this facilitates transfer of the vector prepared in *E. coli* to the second host for production.

In a scenario in which *E. coli* and *Pseudomonas* are used, the vector transmitted between the species should preferably include an appropriate origin of conjugation for *Pseudmonas,* such as *oriT* (Simon et al., 1983, Bio. Technol., 1, 784). The vector should also contain, an origin of replication for maintenance in the first host cell. For example, when the first host cell is *E. coli,* the preferred origin of replication is *oriS* (Birren et al., 1997, in Genome Analysis, a laboratory manual, Cold Spring Habour, Vol 3) in order to give rise to a single copy vector, which increases plasmid stability.

An additional advantage is that, in contrast to the situation in many of the major secondary metabolite-producing hosts, several *E. coli* elements, such as promoters and certain plasmid replication origins, function well in *Pseudomonas* species.

In the final step of the methodology of the invention, the second host cell should be cultured under conditions which are suitable for synthesis of the secondary metabolite. Suitable conditions for growth of the host cell will be known to those of skill in the art. As referred to above, in preferred systems according to the invention, an inducible promoter is used in one or more of the genes that form part of the biosynthetic pathway under study; in these systems, the inducing agent will preferably be added once the host cells have attained a high cell density. This will minimise cell death during earlier stages of growth as a result of potential toxicity of the secondary metabolite produced.

The invention thus incorporates a test for determining whether a secondary metabolite that is toxic for a heterologous host cell is bioactive, by gauging the effect of induction of the complete biosynthetic pathway on the growth of the host cells in which this is taking place.

The transfer of a complete biosynthetic pathway to a second host that does not normally contain that pathway can lead to the accumulation of the expected product of that pathway, but also to the accumulation of novel derivatives of the end product of the pathway, and to novel derivatives of biosynthetic intermediates.

For example, the inventors show that the transfer of the myxochromide S biosynthetic gene cluster from *Stigmatella aurantiaca* to *Pseudomonads pudita* leads to the accumulation of not only myxochromide S, but also to new myxochromide S derivatives that tack the threonine N-methyl group (see examples). These compounds represent novel myxochromide S derivatives and are included herein as aspects of the present invention. Examples of such myxochromide S derivatives are those of formula: 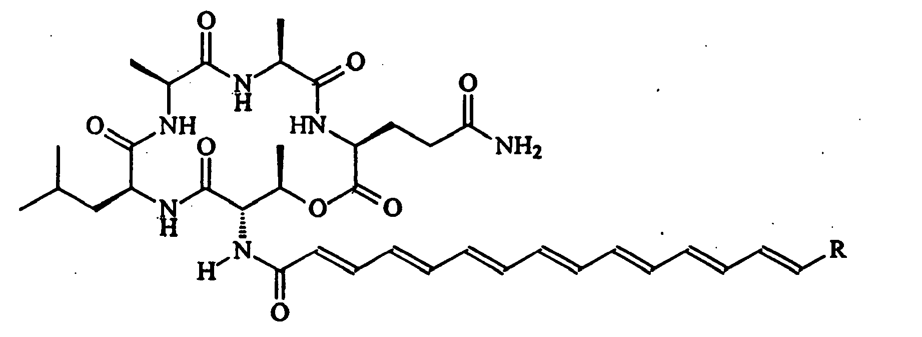 wherein R is selected from the group consisting of CH₃, C₂H₅ and CH=CHCH₃.

The methodology of the invention may be performed iteratively, with successive rounds of screening and selection in order to allow the molecular evolution of one or more of the genes that participates in the pathway toward a desired function. Indeed, an entire pathway can be revolved in this fashion.

For example, the genes encoding the enzymes of the biosynthetic pathway may optionally be further genetically engineered. Mutagenesis of the genes encoding the enzymes is an advantageous way to alter the chemical product because the structure of the secondary metabolite is directed by the specificity of the enzymes of the biosynthetic pathway. Where the secondary metabolite has useful biological properties, genetic engineering of the secondary metabolite preferably alters the biological properties of the secondary metabolite itself, for example, by altering the structure of the molecule generated by the biosynthetic pathway. For example, genetic engineering may enable an increase in the half-life of the secondary metabolite or may increase its specific activity. Where the secondary metabolite is an antibiotic, genetic engineering may for example decrease the IC₅₀ of the antibiotic when compared to the IC₅₀ of the antibiotic synthesised by wild-type enzymes. Furthermore, genetic manipulation may confer a new biological property on the secondary metabolite and/or may delete an existing property. Genetic manipulation of this type may be carried out by shuttling a vector selected in the second host cell back into the first host cell, or may be carried out directly in the second host cell or in a further host cell. As mentioned above, because *Pseudomonas* is quite similar to *E. coli,* certain advances pioneered in *E. coli,* for example, recombineering with RecE/RecT or Redα/Redβ phage proteins, are also potentially applicable to *Pseudomonas.* Thus the use of *Pseudomonas* as the second host cell presents options for *in situ* engineering of pathways after introduction. It is considered possible that in the event that our knowledge of Pseudomonas species is extended, host cells of this type might also be utilised as the first host cell species in the context of the invention described herein.

Because of the relative ease of genetic manipulation in the cloning host, however, it is likely that in most circumstances, genetic manipulation will be effected in the first host cell and then the vector transformed back into the second host cell for screening and selection. The use of a first host in which genetic engineering techniques are well established enables genetic engineering to be carried out with a high degree of accuracy and in particular enables site-directed mutagenesis to be carried out in order to alter the secondary metabolite specifically. Random and/or combinatorial mutagenic approaches may alternatively or additionally be used for the creation of libraries of mutations, including approaches such as DNA shuffling, STEP and sloppy PCR, and molecular evolution. A random and/or combinatorial approach enables libraries of different secondary metabolites to be created.

The genetic engineering of one or more genes in the biosynthetic pathway may involve any suitable type of mutagenesis, for example, substitution, deletion or insertion mutagenesis. If the sequence encoding the one or more genes contains redundant, irrelevant and potentially undesirable sequences, genetic engineering can be carried out to remove these sequences from the vector. Mutagenesis may be carried out by any suitable technique known in the art, for example, by site-directed mutagenesis or by transposon-mediated mutagenesis, as the skilled reader will appreciate. Site-directed mutagenesis may be used to insert new restriction sites, alter glycosylation patterns, change codon preference, produce splice variants, introduce mutations and so forth. Recombineering may also be used where appropriate.

The second host cell may be cultured under any suitable conditions, as will be understood by those of skill in the art. However, it is preferred that the second host cell is cultured between 10°.C and 20°C, for example between 13°C and 18°C. In a particularly preferred embodiment, the second host cell is cultured at 16°C. These culture conditions are particularly preferred when *Pseudomonas* is used as the second host cell. Even more preferably, the second host cell grown at 16°C is *P. putida.*

The invention will now be described further by way of reference so an exemplary system involving expression of the myxochromide gene cluster in *Pseudomonas.* The suitability of *Pseudomonas* as a second host for the production of polyketides and nonribosomal peptides is also illustrated by two examples.

### Brief description of the figures

Figure 1 shows a schematic representation of the generation of pZero-oriT-tet and a restriction digest thereof;
Figure 2 shows the PCR product of the *trpE* gene from *Pseudomonas* together with a schematic representation of the generation of the pZero-oriT-tet-trpE cassette and a restriction digest thereof;
Figure 3 shows a schematic representation of the generation of a subclone of the oriT-tet-trpE cassette in p15A and a restriction digest thereof;
Figure 4 shows a restriction digest of p15A-oriT-tet-trpE together with a schematic representation of insertion of the oriT-tet-trpE conjugation cassette into cosmids containing the myxochromide gene cluster and a restriction digest thereof;
Figure 5 shows the PCR product of the zeocin resistance gene together with a schematic representation of the generation of pMch23 and a restriction digest thereof;
Figure 6 shows a restriction digest of pMch23 together with a schematic representation of the generation of CMch36 and a restriction digest thereof;
Figure 7 shows a schematic representation of the construction of a Pm inducible promoter cassette and a restriction digest thereof;
Figure 8 shows the PCR product of the Pm promoter together with a schematic representation of the insertion of the Pm promoter into the myxochromide S gene cluster and a restriction digest thereof;
Figure 9a, Maps of cosmid E196 and the Red/ET recombinant cosmid CMch34, CMch36 and CMch37 with virtual *Pvu*II restriction, indicated by dotted lines. b, Restriction analysis of cosmid E196 and the RED/ET recombinant cosmid CMch34, CMch36 and CMch37 with the indicated restriction enzymes including *Pvu*II;
Figure 10 shows a gel analysis of the presence of the Myxochromide S gene cluster in transformants;
Figure 11 shows a TLC showing the detection of Myxochromide S production from the mutant *P. putida*/CMch37;
Figure 12a, Structures of myxochromides S₁₋₃ (1-3) produced by the myxobacterium *S. aurantiaca* DW4/3-1. b, HPLC profiles from extracts of the *P. putida*/CMch37 mutant strain in comparison to *P. putida* wild type and the natural myxochromide S producer *S*. *aurantiaca* DW4/3-1 (diode array detection at 200-400nm). Numbers correspond to substances: **1-3** see figure, 4 and 5 are assumed to be the corresponding Des-*N*-methylderivatives from 1 and 3; M, myxothiazol;
Figure 13 shows a combined HPLC chromatogram of extracts from *P. putida* + pFG 136 (negative control for flaviolin, upper trace) and *P. putida* + pFG 154 (rppA expression plasmid, lower trace);
Figure 14 shows the UV-spectrum of peak number 23 of Figure 13, lower trace (P. putida + pFG 154 extract);
Figure 15 shows the positive (+1) and negative (-1) MS spectrum of peak 23, expected mass of flaviolin is 206;
Figure 16 shows the final p15A-sacB-neo-mutase-lacZ-zeo construct for methylmalonyl-CoA production in Pseudomonads;
Figure 17 shows PCR checking results for the integration of methylmalonyl-CoA generation cassette into *P, putida.* The upper panels show the chromosomal region of the P. putida strains harboring the integrated DNA-fragment after double-cross-over event. The letters indicate the primer pairs used for verification in colony-PCR (The schematic diagram in the upper panel is not drawn to scale). The lower panels show the pictures of agarose gels with the amplification products of the corresponding primer pairs;
Figure 18A shows the calibration curve of methylmalonate. The amount ratio is shown on the x axis and the response ratio is shown on the y axis. All calibration samples contained 10 nmol of internal standard methyl-d3-malonate. The methylmalonate quantities were 1,2, 5, 10, 20 and 50 nmol. Each point was measured in triplicate and the data points represent an average of the results. Figure 18B shows the methylmalonate content (a) and OD₆₀₀ (b). The methylmalonate content was calculated from the nmol quantity found in the extract and the OD₆₀₀ at the given time point;
Figure 19 shows the construction of the myxothiazol gene cluster for expression in *P. putida.* Fig. 19A: diagram of strategy for stitching of mta gene cluster. Fig. 19B: restriction result of gene cluster before and after stitching. Fig. 19C: final mta construct for *P. putida* integration;
Figure 20. Figure 20 shows the detection of the myxothiazol in the *P. putida* extracts: a) standard reference myxothiazol A; b) extract of *P. putida* FG2005; peak corresponds to the mass peak of authentic myxothiazol. Figure 20IIshows the mass fragmentation in the reference substance (a) and in *P. putida* FG2005 extract (b);
Figure 21 shows the sequence of operon from So ce56;
Figure 22 shows the best 20 Hits from a search of the database with BLASTP with methylmalonyl-CoA mutase of *Sorangium cellulosum* So ce56 as input and alignment of top 2 scores with the query sequence;
Figure 23 shows the best 20 Hits from a search of the database with BLASTP with methylmalonyl-CoA epimerase of *Sorangium cellulosum* So ce56 as input and alignment of top 3 scores with the query sequence;
Figure 24 shows the best 20 Hits from a search of the database with BLASTP with MeaB of *Sorangium cellulosum* So ce56 as input and the alignment of the top 2 scores with the query sequence;
Figure 25 shows the MycoMar transposase DNA and protein sequences and its inverted repeat sequences;
Figure 26 is a diagram of *myxochromide S* gene cluster engineering. Fig. 26A shows the insertion of MycoMar transposase gene plus right *IR* into *mchS* expression plasmid and Fig. 26B shows the integration of the left *IR* plus *Tn5-neo* gene in front of the *mchS* gene cluster;
Figure 27A shows the detection of *myxochromide S* compounds in *M. xanthus.* Figure 27B shows the detection of *myxochromide* compounds in TCL; and
Figure 28A shows HPLC peaks obtained from analysis of a methanol extract from an *M. xanthus* DK1622:*mchS* mutant strain for the production of myxochromides S. (Figure 28B shows the UV spectra from each of peaks 1-7 of the HPLC chart resulting from the characteristic myxochromide chromophores.
Table 1 shows MALDI /TOF results summary of the *Pseudomonads* PPant transferase activity evaluation.
Table 2 shows the number of transformants obtained using transposon-mediated integration compared to the number obtained using homologous integration.

### Examples

### Example 1:

### Synthesis of the type I polyketide/nonribosomal peptide myxochromide in P. putida

The invention is described below in an example in which a compute myxobacterial pathway for the synthesis of the type I polyketide/nonribosomal peptide myxochromide is engineered in E. *coli* and then transferred to *Pseudomonas putida* by conjugation, using a BAC or cosmid vector comprising an *oriT* conjugation region.

### A. Engineering of pSuperCos-Myxochromide to introduce the conjugation origin and tetracycline inducible regulon.

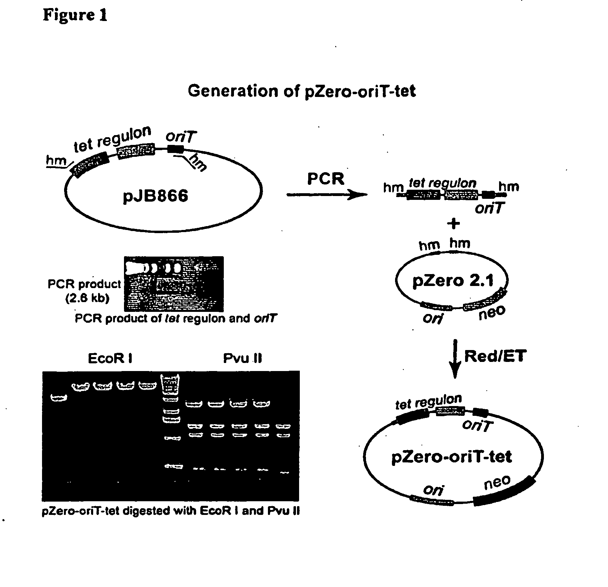

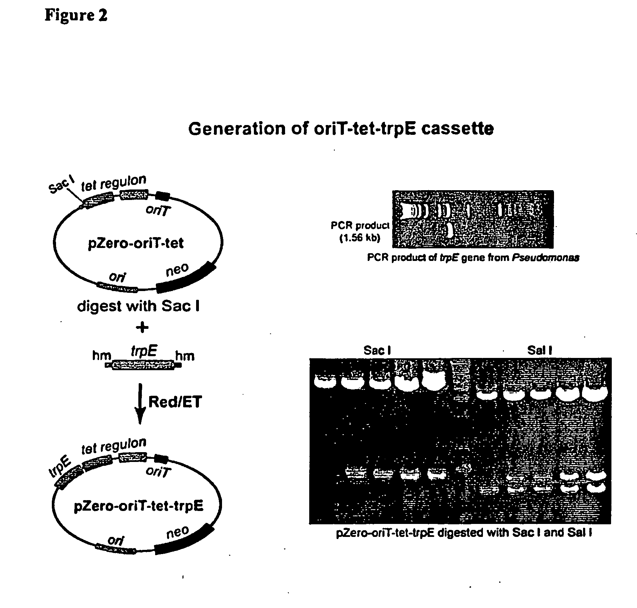

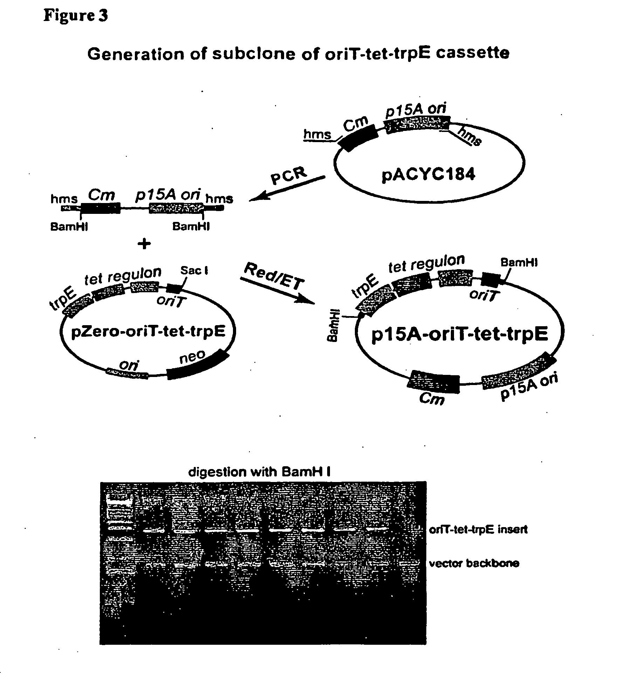

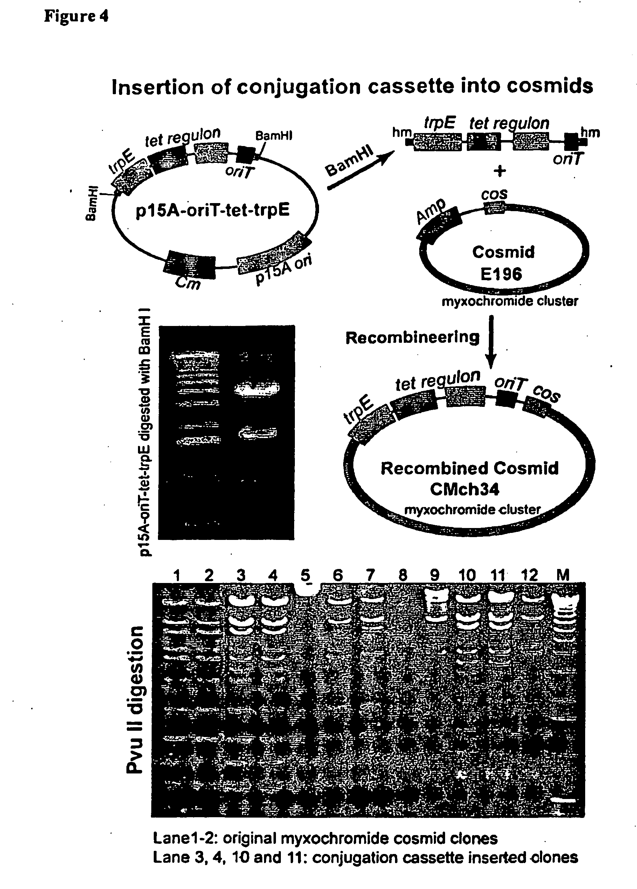

PCR was used to generate an oriT-tetR fragment. *oriT* is the sequence used for conjugation between bacterial species. *TetR* is a tetracyline regulon and consists of the *tet* regulator and the *tet* resistant gene. The oriT-tetR fragment was inserted into the pZeo2.1 vector (Invitrogen) by recombineering (Figure 1). Next, the *trpE* gene from *Pseudomonas* was inserted into the oriT-tetR cassette using recombineering (Figure 2). The *trpE* gene is in this instance used as homology for homologous recombination in *Pseudomonas.* Homology arms for recombineering the oriT-tetR-trpE cassette into the pSuperCos (Stratagene) vector backbone were added in one recombineering step by subcloning into a p15A ori plasmid (Figure 3). The oriT-tetR-trpE cassette was then inserted into the vector backbone part of pSuperCos-Myxochromide by recombineering (Figure 4).

### B. Reconstruction of the complete myxochromide pathway.

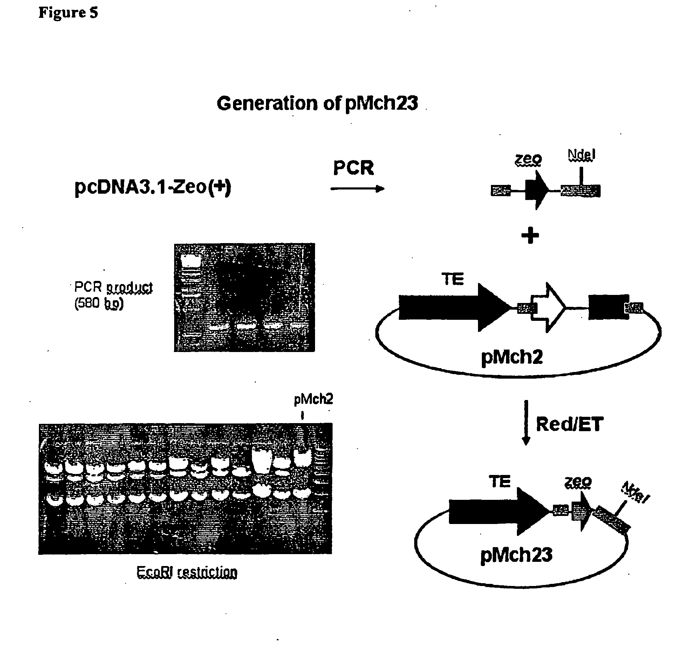

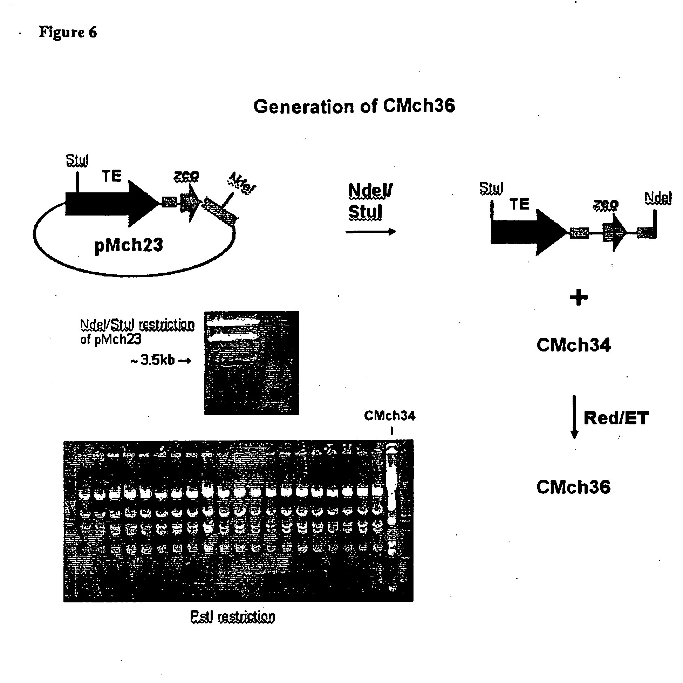

The myxochromide S biosynthetic gene cluster has been cloned and sequenced. The original cosmid E196 does not contain the full-length pathway because it is missing the thioesterase (TE) domain of the second NRPS. To complete the myxochromide S biosynthetic gene cluster and add the necessary elements for conjugation, integration and expression in pseudomonads, the original cosmid E196 was modified sequentially by recombineering using Redact recombination. In brief, the backbone of cosmid E196 was modified by single step insertion of the origin of transfer *(oriT)* for conjugation purposes, the tetracycline resistance gene for selection in *P. pudita* and a DNA fragment from the chromosome of *P. pudita* (*trpE*), to enable the integration of the construct into the genome by homologous recombination, to create the SuperCos derivative CMch34 (Figure 4). During this procedure, the original ampicillin resistance gene of SupetCos was deleted. To reconstruct the complete myxochromide S pathway on CMch34, the missing part of the TE domain had to be added. The sequence of the full-length TE domain was available on pSWMch2, a previously described recovery plasmid from a NRPS mutant strain. To stitch the missing thioesterase piece of the gene cluster onto CMch34, the zeocin resistance gene *(zeoR)* was amplified by PCR reaction and inserted into pSWMch2 by recombineering resulting in plasmid pMch23 (Figure 5). Then, a 3.5 kb Stul/Ndel fragment from pMch23 containing the TE-zeoR cassette was recombined with CMch34 to create CMch36 (Figure 6).

### C. Generation of Pm promoter cassette and insertion of the inducible Pm promoter in front of the Myxochromide gene cluster

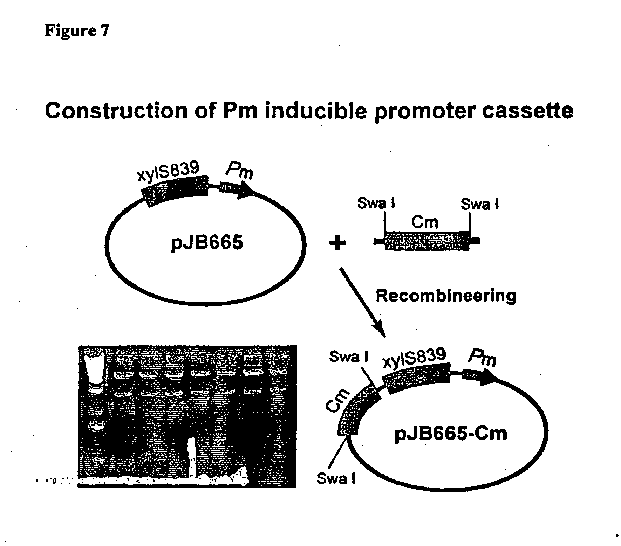

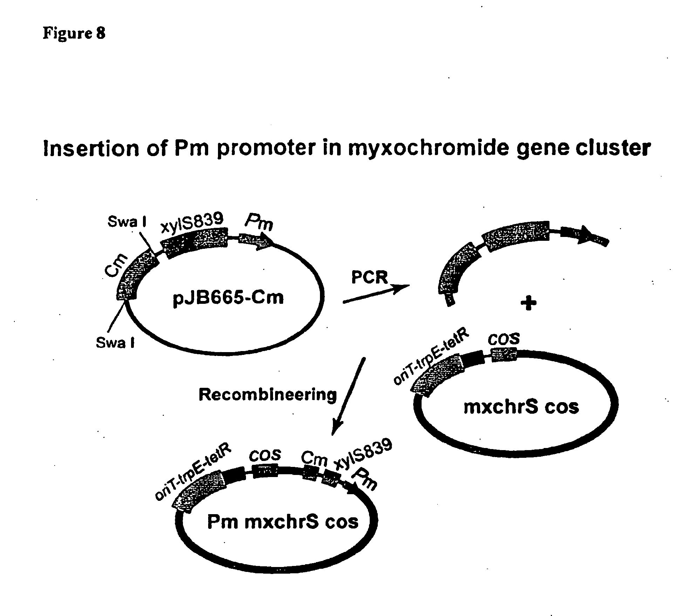

As a final step the toluic acid inducible Pm-promoter was inserted in front of the first gene of the myxochromide S cluster. Together with the chloramphenicol resistance gene and the *xylS* gene, the Pm-promoter was inserted into CMch36 to create CMch37. This insertion was designed to not only place the promoter directly in front of the PKS but also to delete five genes not involved in myxochromide S biosynthesis (Figure 7). The final construct CMch37 contains only the three genes from the myxochromides S pathway (one PKS and two NRPSs) with the Pm-promoter placed in front of the PKS (Figure 8).

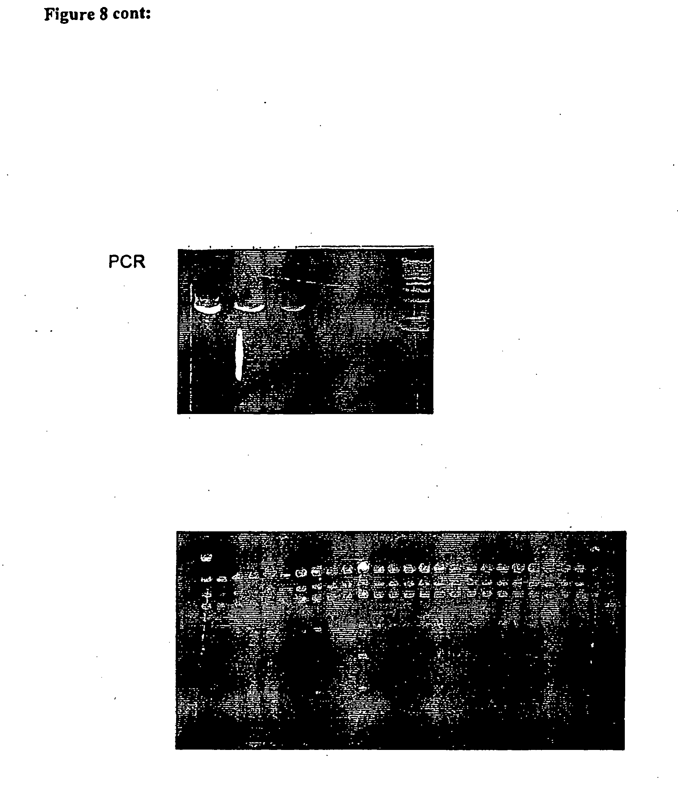

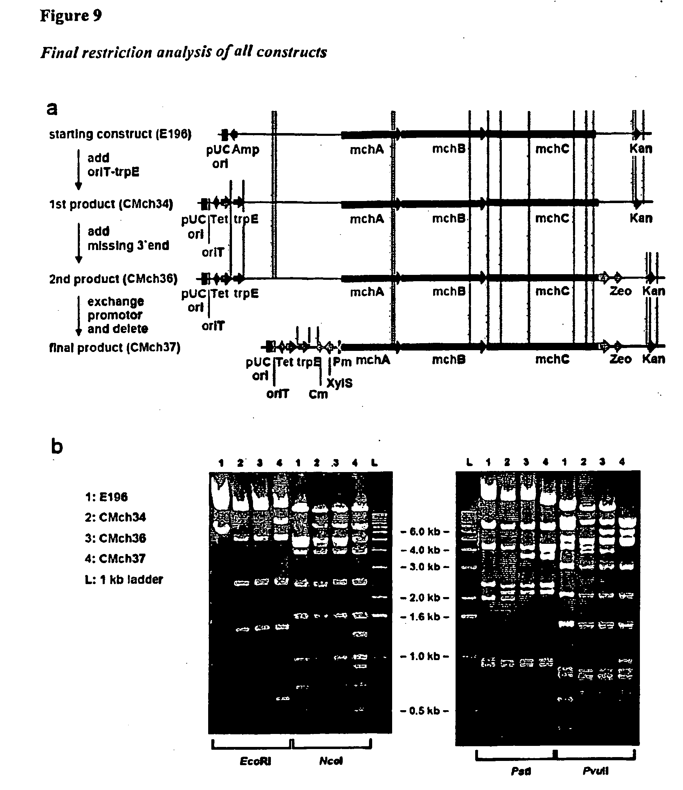

A restriction analysis of the various constructs used in these Examples is shown in Figure 9.

### D. Conjugation of the final construct into Pseudomonas

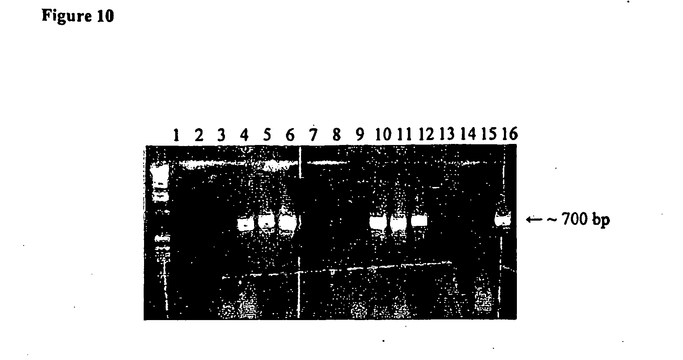

Three *Pseudomonas* strains were used for conjugation (*P. pudita* KT2440, *P. stutzeri* Dim10701, *P. syringae* pv. tomato DC3000). In this particular experiment, only *P. putida* acquired the *mxchrS* gene cluster. After the conjugation, the presence of the Myxochromide gene cluster in the transformants was analyzed by colony PCR (using primers for the amplification of a ca. 700bp fragment from the NRPS2 gene). The results are shown in Figure 10. The various transformants were as follows: Lanes 1, 7 and 13: *P. putida* wildtype; Lanes 4 and 10: *P. putida*/pCMch37; Lanes 2, 8 and 14: *P. stutzeri* wildtype; Lanes 5 and 11: *P. stutzeri*/pCMch37; Lanes 3, 9 and 15: *P. syringae* wildtype; Lanes 6 and 12: *P. syringae*/pCMch37; Lane 16: Cosmid E196. The results show that the strains transformed with pCMch37 contain the 700bp sequence, which is also present in the cosmid pE196.

### E. Production and detection of myxochromide S in Pseudomonads

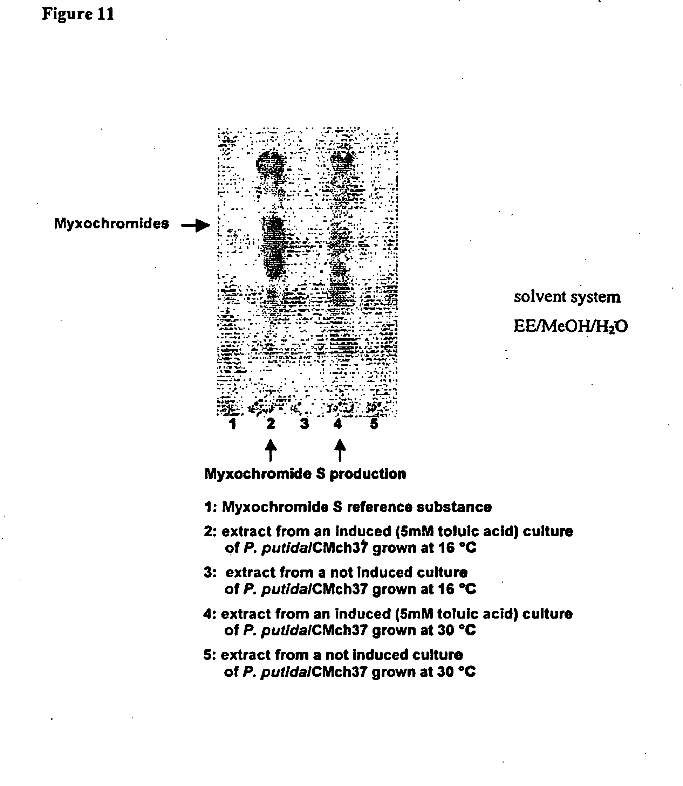

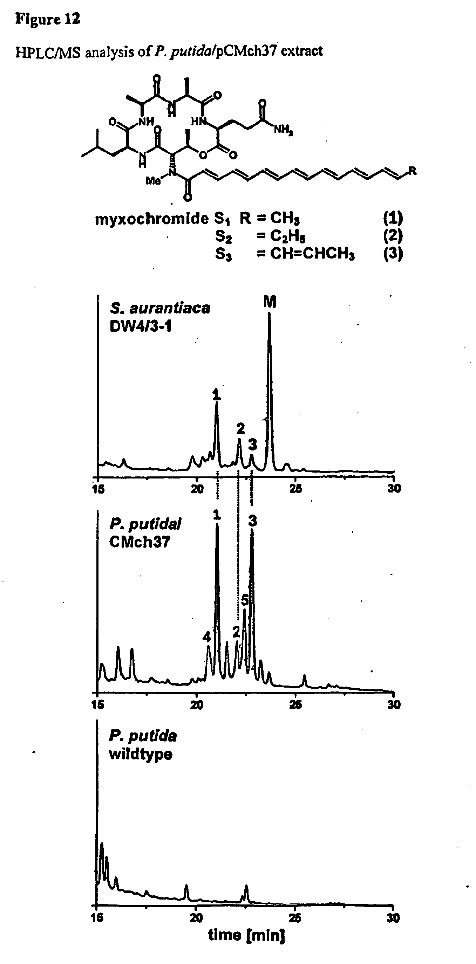

To induce expression from the Pm-promoter in *Pseudomonas* strains currying the complete myxochromide S biosynthetic gene cluster, toluic acid was added to cultures after two hours of fermentation. After induction myxochromide S could be detected by TLC (Figure 11). Compared to 30°C, cultivation at 16°C after the induction resulted in a more than 1000-fold increase of myxochromide S production reaching a maximum yield of approximately 40 mg/l. This is about 5 times greater than the maximum found with the natural producing host, *S. aurantiaca.* Furthermore, new myxochromide S derivatives could be identified in these extracts by HPLC/MS analysis (Figure 12). In addition to myxochromides S1-3, known from S. *aurantiaca,* the MS data from extracts indicate the presence of the corresponding compounds lacking the threonine N-methyl group and thus representing new myxochromide S derivatives. Myxochromide were only detected in the cells and not in the fermentation medium, indicating that *P. pudita* is not able to export these secondary metabolites out of the cell. A kinetic of myxochromide S production in *P. pudita*/CMch37 mutants reveals that the production maximum was reached after 2-3 days, which surpasses the 6 days required for *S. aurantiaca* to reach maximum production.

### Example 2:

### Pseudomonas is able to express Type III PKS

### A) Introduction

In the course of the ongoing genome sequencing project of *Sorangium cellulosum* So ce56 homology searches with the BLAST program were performed. An open reading frame was identified, which shows homology to type III polyketides from bacteria. The encoded protein has about 70% identity with the 1,3,6,8-tetrahydroxynaphtalene synthase (RppA) from several streptomycetes. This enzyme is responsible for the production of 1,3,6,8-tetrahydroxynaphtalene, which oxidises spontaneously to flaviolin. From the extent of homology to RppA, it could be assumed that the product of the reaction catalysed by this enzyme would be 1,3,6,8-tetrahydroxynaphtalene or flaviolin, respectively. Such a compound is undetected to date in *Sorangium cellulosum* So ce56, although the screening program performed with this strains was extensive. The compound has not been found in any myxobacterium. The assumption is that the corresponding gene is silent in the wild type.

### B) Construction of the expression plasmid

The corresponding gene was amplified by PCR and the fidelity of the amplicon verified by nucleotide sequencing. The gene was cloned to generate a C-terminal intein-chitin binding domain fusion and subsequently subcloned into a broad host range vector based on RK2 to allow independent replication in *Pseudomonads.* The final construct (pFG154) was transferred by conjugation into *Pseudomonas putida.*

### C) Detection of flaviolin production from P. putida+pFG154

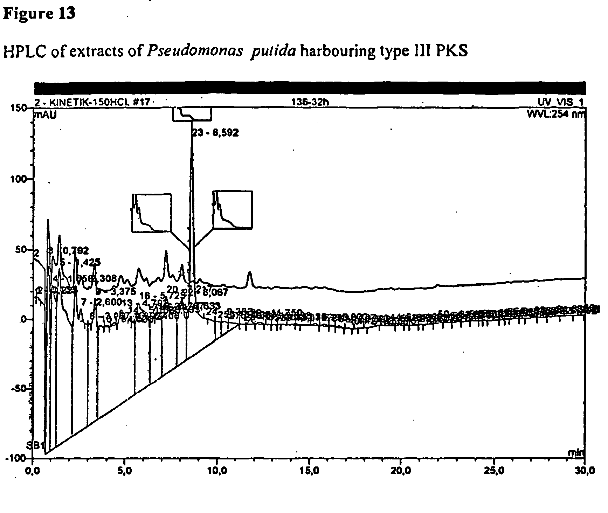

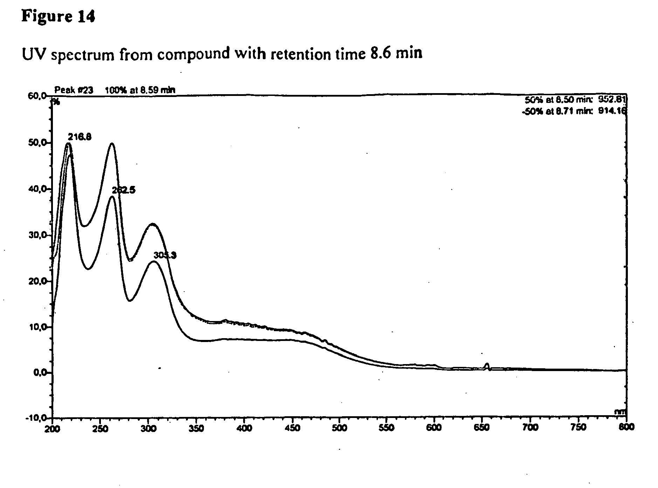

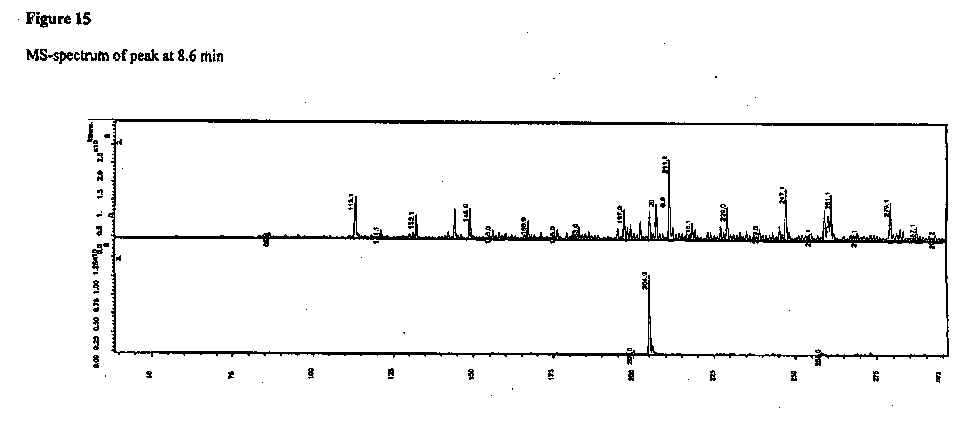

*Pseudomonas putida* harbouring plasmid pFG154 was cultivated, harvested after 32 hours and the culture supernatant after acidification extracted with ethyl acetate. The organic solvent was completely evaporated and the residue was dissolved in methanol. The methanolic extract was subjected to HPLC analysis (Figure 13). The flaviolin compound produced by *P. putida*+pfG154 was confirmed by its UV-spectrum (Figure 14) and HPLC-MS (Figure 15). The compound was also confirmed as flaviolin by NMR.

### Example 3:

### Evaluation of Pseudomonas strains for PPANT transfersase activity

We demonstrated the ability of *Pseudomonas putida* KT2440, *Pseudomonas syringae* pv. *tomato* DC3000 and *Pseudomonas stutzeri* DSM10701 to posttranslationally activate carrier protein domains of polyketide synthases, nonribosomal peptide synthetases and fatty acid synthase by their intrinsic phosphopantetheinyl transferase. The apo-form is modified to the holo-form of the carrier protein through attachment of a phosphopantetheine moiety from coenzyme A to a conserved serine residue of the carrier protein (domain). We cloned the coding region of the respective domains in order to generate C-terminal fusions with intein-chitin binding domain. The constructs were subcloned into a broad host range vector and transferred into the three *Pseudomonas* hosts. Resulting recombinant *Pseudomonas* strains were cultivated and each fusion protein was purified by affinity chromatography.

The purified carrier protein was analysed using MALDI/TOF for a mass increase of 340 mass units expected to be the phosphopantetheine moiety. From the carrier proteins tested, six could be purified from *Pseudomonas putida,* which was chosen as the general host. Out of the six domains five were completely activated, whereas of the sixth domain only 5% of the protein was in the holo-form. Four domains were also expressed in the other alternative hosts. The MALDI/TOF analysis results are shown in Table 1.

### Example 4:

### Production of methylmalonyl-CoA in Pseudomonads

### A) Cloning of genes from Sorangium cellulosum for methylmalonyl-CoA production.

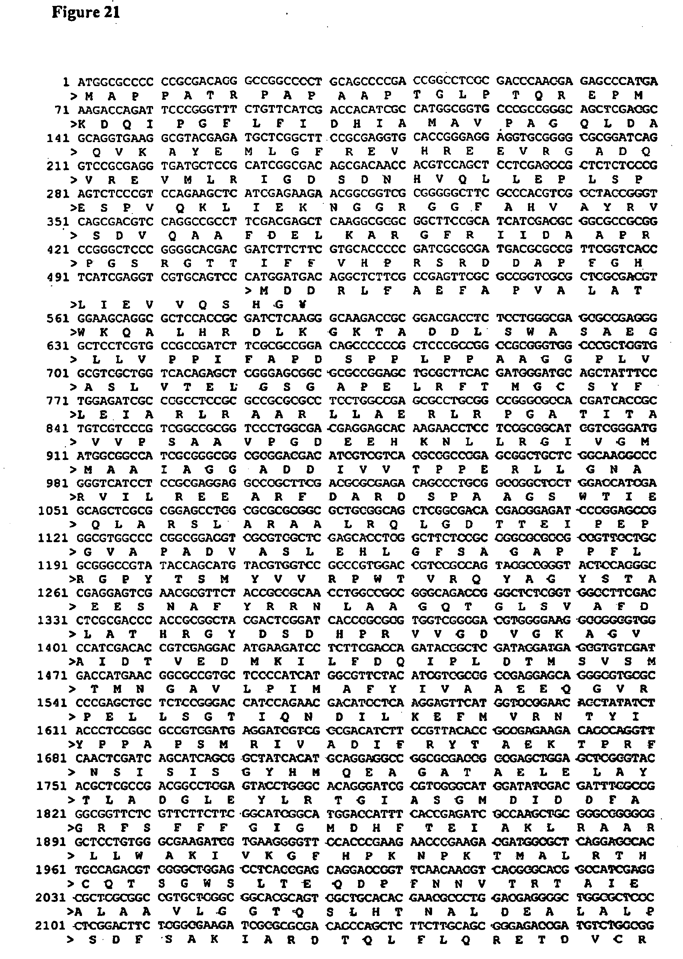

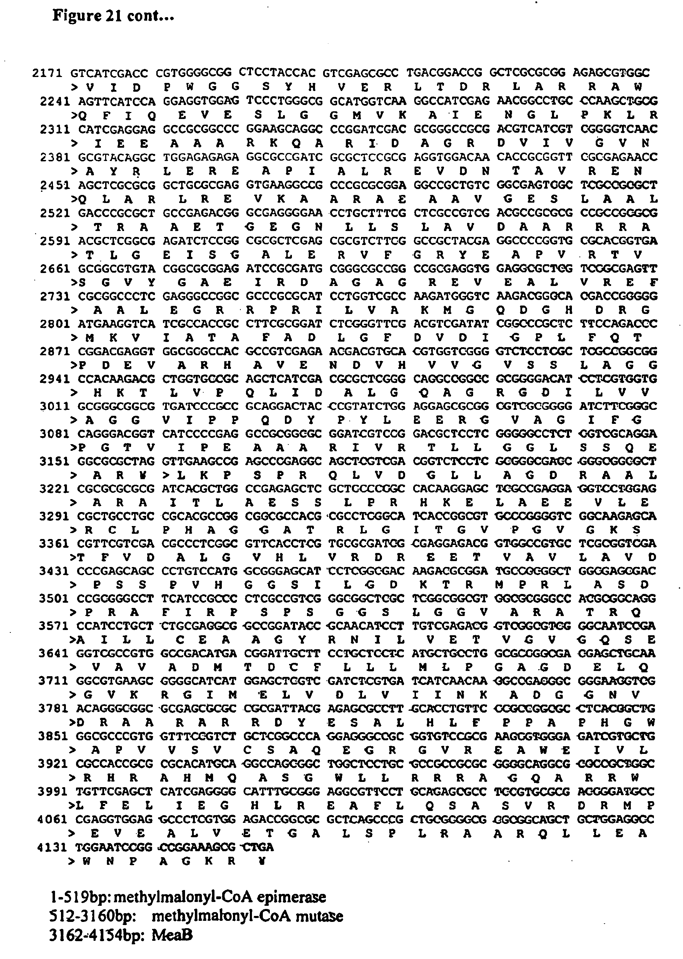

To accomplish the task of hetero-expression of all possible polyketide gene clusters in Pseudomonads, foreign genes encoding the peptides to synthesize methylmalonyl-CoA may be integrated into Pseudomonas strains. An operon from *Sorangum cellulosum* So ce56 (So ce56) is predicted to encode the enzymes for methylmalonyl-CoA production from succinate. The methylmalonyl-CoA epimerase (*epi,* sce_20050509_2546), methylmalonyl-CoA mutase (mcm, sce_20050S09_2547) and *meaB* (sce_20050509_2548) were identified *in silico* by homology searches with the BLAST software. The sequence of the operon from So ce56 is shown in Figure 21.

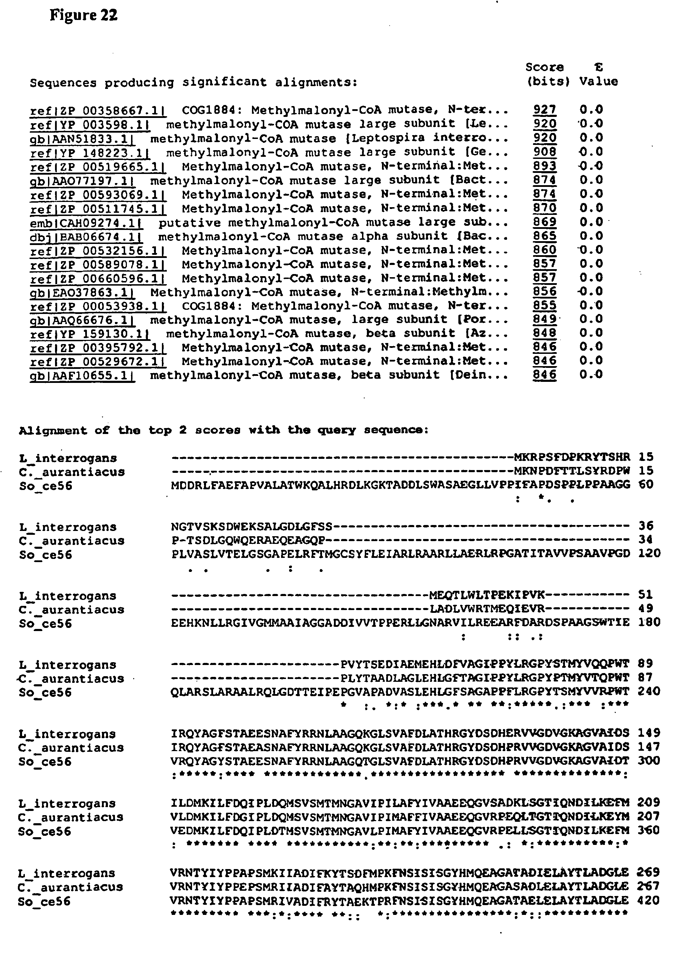

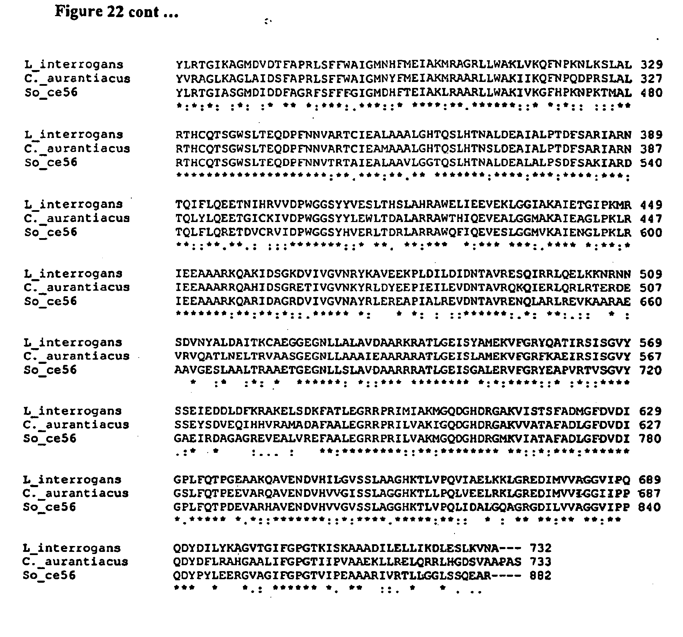

The open reading frame of the predicted methylmalonyl-CoA mutase is 2649 nucleotides long. The blast results and alignments of Figure 22 show that the deduced protein (882 amino acids) shows highest homologies to the methylmalonyl-CoA mutases of *Chloroflexus aurantiacus* (ZP_00358667; identities: 460/670 (68%), positives: 537/670 (80%)) and *Leptospira interrogans* serovar Copenhageni str. Fiocruz L1-130 (YP_003598; identities: 460/677 (67%), positives: 539/677 (79%)).

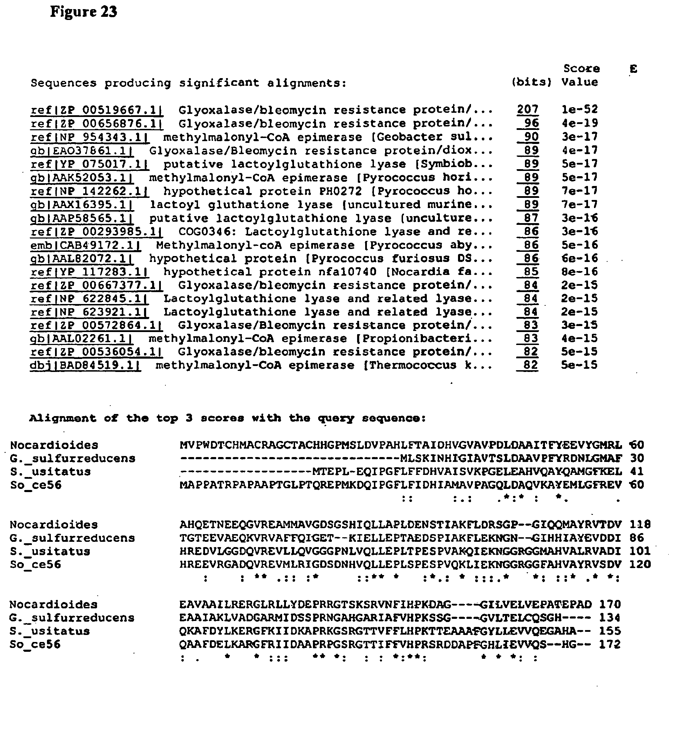

The predicted methylmalonyl-CoA epimerase is 519 nucleotides long. The blast results and alignments of Figure 23 show that the deduced protein (172 amino acids) shows highest homologies to predicted glyoxylases/bleomycin resistance genes (lactoylglutathione (LGSH) lyases family) from *Solibacter usitatus* Ellin6076 (ZP_00519667; identities: 98/149 (65%), positives: 125/149 (83%)) and *Nocardioides* sp. JS614 (ZP 00656876; identities: 58/151 (38%), positives: 87/151 (57%)) and then to a predicted methylmalonyl-CoA epimerase from *Geobacter sulfurreducens* PCA (NP_954343; identities 59/139 (42%), positives: 83/139 (59%)).

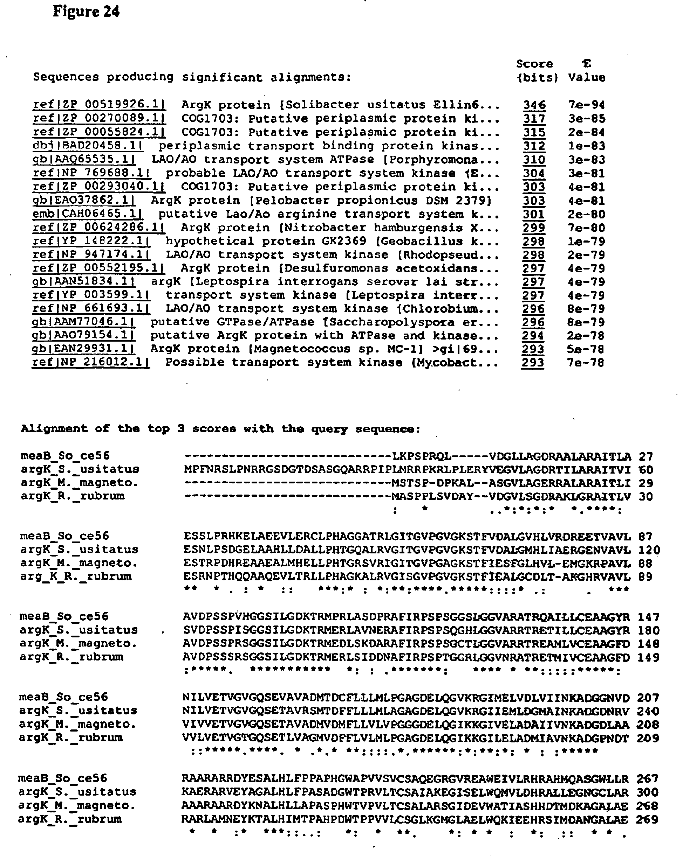

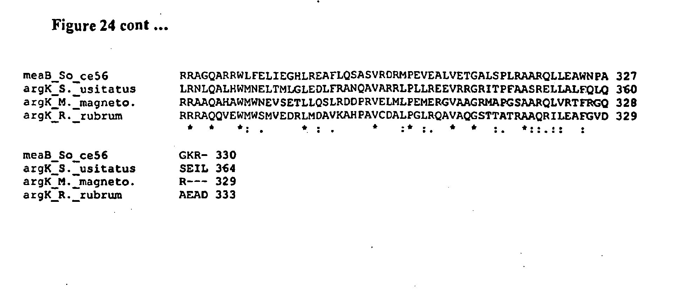

The open reading frame of *meaB* is 993 nucleotides long. The blast results and alignments of Figure 24 show that the deduced protein (330 amino acids) shows highest homologies to *argK* (lysine/arginine/ornithine (LAO) transport protein family) from *Solibacter usitatus* Ellin6076 (ZP_00519926; identities: 183/298 (61%), positives: 218/298 (73%)).

It was proposed that the annotation of homologues to LGSH lyases and LAO transport proteins, which are clustered with methylmalonyl-CoA mutase, are misidentified by homology searches (Haller et al., 2000; Bobick & Rasche, 2001) and that they actually belong to the propionyl-CoA. metabolism towards succinyl-CoA via methylmalonyl-CoA.

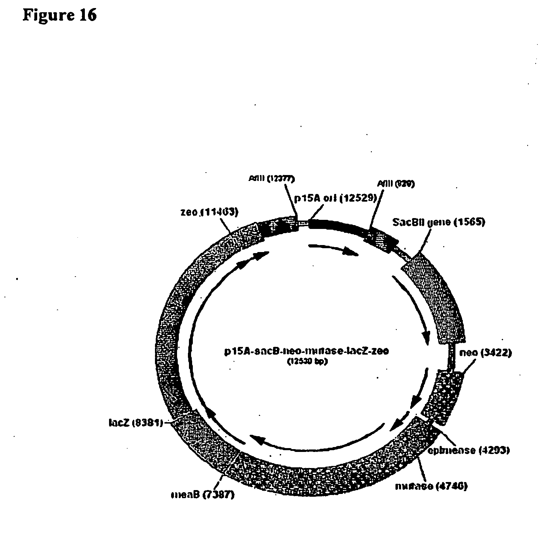

The operon-containing BAC clone generated from genomic DNA of So ce56 was used for downstream experiments. The final integration cassette in a p15A origin based plasmid is shown in Figure 16. After subcloning the operon into a p15A origin based plasmid, sacB-neo, a counter-selection cassette, was placed in front of the epimerase gene to drive the operon expression and lacZ-zeo was placed at the end of operon (end of meaB gene) for reporting the Tn5 promoter which drives 6 genes. Two homology arms generated from PCR products of trpE gene from P. putida were cloned at both ends of sacB-neo-epi-mut-meaB-lacZ-zeo for homologous integration into Pseudomonas putida. To shorten the plasmid name, the final construct was called p15A-sacB-neo-mutase-lacZ-zeo. A ribosomal binding site was placed in between each of the Tn5-neo and epimerase genes, the meaB and lacZ genes, and the lacZ and zeocin resistant genes. All of the steps were done by using Red/ET recombination.

### B) Integration of methylmalonyl-CoA generation cassette into Pseudomonas putida.

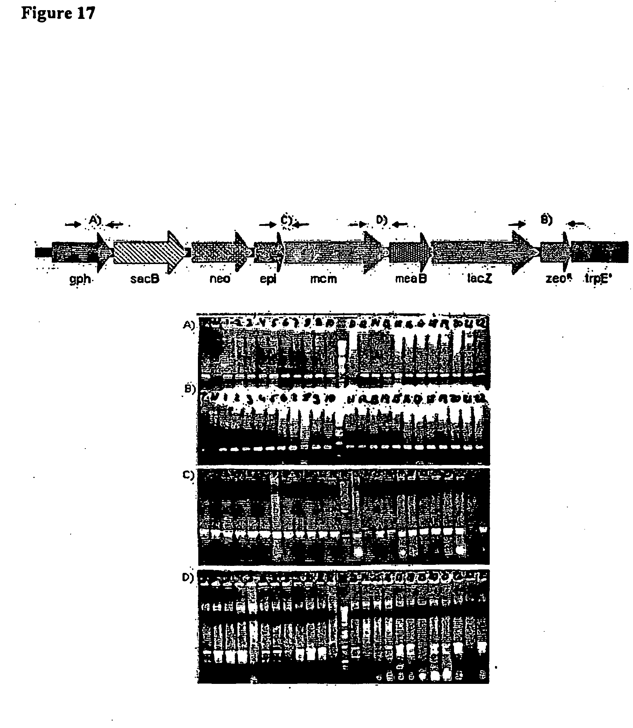

The expression plasmid p15A-sacB-neo-mutase-lacZ-zeo was transformed into *P. putida* by electroporation and kanamycin resistant atones were selected. The electrocompetent cells were prepared as follows: 1.4 ml LB medium in a 1.5 ml reaction tube were inoculated with 30 µl of a saturated overnight culture of *P*: *putida* KT2440 and incubated for 2 hours at 28°C with shaking. The cells were washed twice with ice cold water and resuspended after the last washing step after pouring off the water in the remaining liquid. 1 µl of a plasmid minipreparation of p15A-sacB-neo-mutase-lacZ-zeo was added and the cell suspension transferred to a 1 mm electroporation cuvette. The cells were pulsed with a voltage of 1.1 kV in an Eppendorf electroporator 2510, then 500 µl LB medium were added, the cells transferred to an 1.5 ml reaction tube and incubated for 60 min at 30°C with shaking for phenotypic expression. The transformed cells were spread on a LB agar petri dish containing 15 µg/ml of kanamycin and incubated at 30°C overnight. To further verify whether the clones are intact in *P. putida* chromosome, primers were used for colony-PCR reaction. Referring to Figure 17, the primers used for panel A PCR reaction were 5'-GGACCAGATGAAGATCGGTA-3' and 5'-TGTTCATCGTTCATGTCTCC-3'; Primers used for panel B PCR reaction were 5'-CGACTTCCAGTTCAACATCA-3' and 5'-GATTCGAGCAGGTACGAGTT-3'; Primers used for panel C PCR reaction were 5'-GCTTCGCCCACGTCGCCTACC-3' and 5'-CGACGATGCCGCGGAGGAGGTT-3'; Primers used for panel D PCR reaction were 5'-CGAGACGGGCGAGGGGAACC-3' and 5'-CGTCTTGTCGCCGAGGATGCT-3'. PCR checking results are shown in Figure 17.

### C) Detection of methylmalonyl-CoA in engineered P. putida strains,

### Methods

### C-i) Sample preparation for GC

Methylmalonate was transformed into its butyl ester by a procedure based on the method of Salanitro and Muirhead (Salanitro, J.P. and Muirhead, P.A., 1975, "Quantitative method for the gas chromatographic analysis of short-chain monocarboxylic and dicarboxylic acids in fermentation media." Appl. Microbiol. 29(3): 374-81). An aliquot (300 µl) of cell extract was transferred to a glass vial (1.8 ml), 10 nmol of the internal standard methyl-d3-malonic acid were added, and the mixture was evaporated to dryness in a vacuum concentrator. To the dry sample, we added 400 µl of hexane and 100 µl of HCI in 1-butanol. The vials were capped with teflon-lined screw caps and incubated at 80°C for 2 h. After cooling down to room temperature, the reaction mixture was neutralized with 500 µl of an aequous solution of Na₂CO₃ (6% *m*/*V*), and the vials were centrifuged to achieve complete phase separation. The upper organic phase was injected into the gas chromatograph. All quantitations were carried out in duplicate unless otherwise stated.

### C-ii) Gas chromatography - mass spectrometry

Samples were measured on an Agilent 6890N gas chromatograph equipped with a 5973N mass selective detector and a 7683B automatic, liquid sampler. The stationary phase was a HP-5ms capillary column (0.25 mm x 30 m x 0.25 µm, Dimethylpolysiloxane with 5 % phenyl rests), and the carrier gas was helium at a flow rate of 1.5 ml/min. The temperature gradient used was as follows: 70°C 5 min isothermal, heating up to 170°C at 5 °C/min, heating up to 300°C at 30°C/min, 300°C 5 min isothermal, then cooling down to 70°C at 30°C/min. A pulsed splitless injection mode was used injecting 2 µl of sample. For quantitation, the mass detector was configured for single ion monitoring (SIM), scanning ions m/z 101, 104 and 105 at a dwell time of 100 ms per ion, and the quantitation was based on the ratio of the areas of the ions *m*/*z* 101 (Methylmalonate) and m/z 104 (Methyl-d3-malonate). Calibration was done by injecting triplicate samples of 1, 2, 5, 10, 20 and 50 nmol methylmalonate with 10 nmol methyl-d3-malonate in each sample. Data analysis, calibration and quantitation were carried out with Agilent ChemStation software.

### Results

### C-iii-) GC/MS Calibration

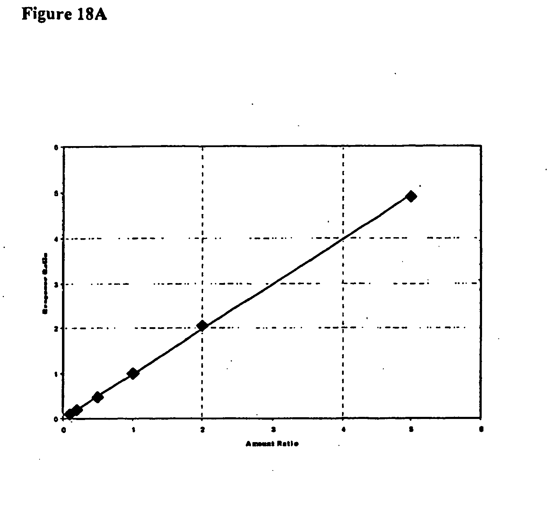

Methylmalonate showed linearity over the full concentration range (r² = 0.999). However, the best fit was obtained using the average response factor of 0.991+/-3.1% instead of linear regression. Recovery was 100+/-4%. The calibration curve is shown in Figure 18A. All calibration samples contained 10 nmol of internal standard methyl-d3-malonate. The methylmalonate quantities were 1, 2, 5, 10, 20 and 50 nmol. Each point was measured in triplicate.

### C-iv) Quantitation of Methylmalonate

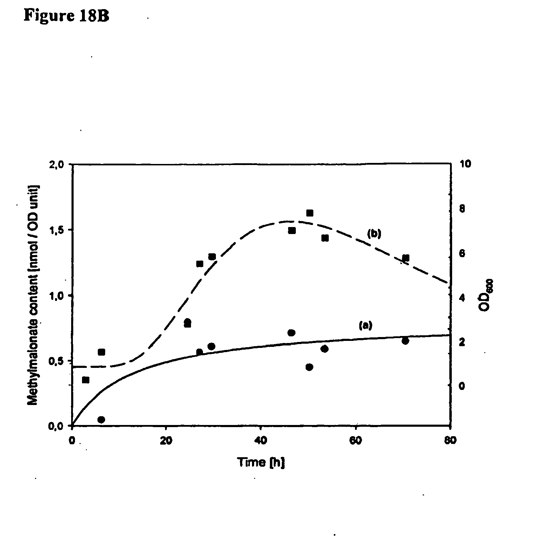

The methylmalonate content was calculated from the nmol quantity found in the extract and the OD₆₀₀ at the given time point, and the results are shown in Figure 18B (methylmalonate content (a) and OD₆₀₀ (b)). After 24h, methylmalonate could be determined in *P. putida* FG2005, and methylmalonate content remained almost constant from that time point on. The wild type did not show any significant methylmalonate quantities.

### D) Engineering and hetero-expression of myxothiazol (mta) gene cluster

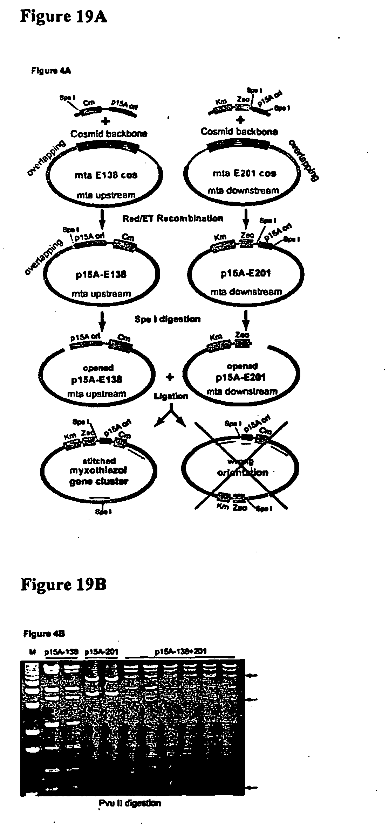

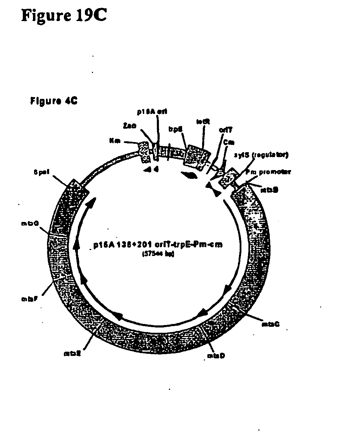

Production of myxothiazol from its gene cluster must utilize methylmalonyl-CoA. Hetero-expression of myxothiazol gene cluster in engineered *P. putida* FG2005 strain is used to evaluate the methylmalonyl-CoA production. Unfortunately myxothiazol gene cluster is presented in 2 cosmids. To obtain the full gene cluster in one vector, several steps of engineering were carried out using Red/ET recombination as represented schematically in Figure 19. A diagram of strategy for stitching of mta gene cluster is shown in Figure 19A. The mta gene cluster is presented in two cosmids. One of the fragments was subcloned into the *p15A-Cm* minimum vector and another one was subcloned into the *p15A-Km-Zeo* minimum vector. At the same time, a *Spe I* restriction site was inserted into the sites for stitching. After subcloning of each fragment into *p15A* origin based vectors using Red/ET recombination, both recombinants were digested with *Spe I* and the fragments were ligated to form the stitched full-length gene cluster. The results of the restriction of the gene cluster before and after stitching are shown in Figure 19B. Ligation products are at both orientations. The right clones with stitched gene cluster are shown in Figure 19B with *Pvu II* digestion. Junction regions of stitched construct were verified by sequencing as well. The final mta construct for P.putida integration is shown in Figure 4C. After stitching, the final construct (Figure 19C) was generated from the stitched construct using two further modifications involving Red/ET recombination. At first, the *tetR-trpE-oriT cassette* was used to replace the *Cm* gene in the stitched construct. This cassette will be used for conjugation and integration into *P. putida.* To regulate *mta* gene cluster expression in *P. putida,* a toluolic acid inducible *Pm* promoter plus its regulator gene and *Cm* selectable gene were inserted in front *of mta* B gene (first module of *mta* gene cluster).

### E) Production of myxothiazol in P. putida FG2005.

### Methods

### E-i) Conjugation of myxothiazol gene cluster into Pseudomonads

The engineered and stitched myxothiazol gene cluster in p15A 138+201 oriT-trpE-Pm-cm was introduced into the chromosome of the *P. putida* KT2440 wild-type as well as into the chromosome of the methylmalonate-generating *P. putida* FG2005 by tri-parental conjugation using helper plasmid pRK2013 (Figurski, D.H., and Helinski, D.R., 1979, "Replication of an origin-containing derivative of plasmid RK2 dependent on a plasmid function provided in trans". Proc. Natl. Acad Sci. U.S.A., 76:1648-1652). 1.5 ml of overnight cultures of *E.coli* HB101 containing plasmid with myxothiazol gene cluster, *E. coli* HB101 harbouring pRK2013 and *P*. *putida* were harvested and resuspended in 300 µl LB medium. 50 µl of each suspension were mixed and dropped onto the LB agar plate. After incubation at 37°C for 4 h the plate was transferred to 28°C and incubated overnight. Then the cells were scraped from the plate, resuspeded in 100 µl sterile water and plated onto the selection PMM agar plates containing either tetracycline (25 µg/ml) for selection for the cosmid with the myxothiazol biosynthetic genes or tetracycline and kanamycin (50 µg/ml) to perform selection for the clones containing myxothiazol genes in the methylmalonate producing *P. putida* FG2005. The obtained clones were tested by colony PCR using myxothiazol specific primers designated for different parts of the gene cluster to verify the integration of the whole biosynthetic gene cluster into the chromosome. The primers used for checking *mtaB* gene are '5' -gaacgtggtcgtctcgggag- 3' and 5'- cgaatcaccagcccggagac- 3'; for checking *mtaE* gene are 5' - tcaagccggatgaggtctac - 3' and 5'- cttggacacggtatcgaggt - 3'; for checking *mtaG* gene are 5' - ctcttcttcatgcatccgac- 3' and 5'- ccggtacatctgaacctgct- 3'.

### E-ii) Extraction for methylmalonate detection

100 ml LB supplemented with 50 µg/ml kanamycin were inoculated with 1:1000 diluted overnight culture of *P. putida* FG2005, incubated at 30°C on a rotary shaker (180 rpm) and harvested at different time points as shown in Figure 3B. The cells were collected by centrifugation. The cell pellets were frozen in liquid nitrogen, then thawed on ice, resuspended in PBS buffer and the cell lysates were prepared using French Press. After addition of 50 ml methanol, the suspension was incubated for 1h with agitation and then filtered through a folded paper filter. The methanol was removed *in vacuo* and the residue dissolved in 1 ml methanol.

### E-iii) Analysis of the heterologous myxothiazol production in P. putida

The *P. putida* strain producing methylmalonate and containing the myxothiazol biosynthetic gene cluster integrated into the chromosome was inoculated with overnight culture (1:140) and incubated in 300 ml flasks containing 50 ml LB medium supplemented with tetracycline (25 µg/ml) and with 2 % of XAD 16 for 1-2 h at 30°C with shaking. The myxothiazol production was induced with toluic acid (5 mM) and the culture was transferred to 16°C and incubated for 2 - 3 days. The cells were harvested by centrifugation and extracted with acetone and methanol. The extracts were evaporated and resuspended in 1 ml methanol. 5 µl of the extracts were analyzed by LC-MS. The chromatographic conditions used were as follows: RP column Nucleodur C18, 125 x 2 mm, 3 µm, and pre-column C18, 8 x 3 mm, 5 µm. Solvent gradient (using solvent A and B with solvent A being water and 0,1% formic acid, and solvent B being acetonitrile and 0,1% formic acid) from 5% B at 2 min to 95% B within 30 min followed by 4 min with 95% B. The mass was detected in positive ionization mode. The myxothiazol A was identified by comparison to the retention times and the MS data of the reference substance ({M+H]⁺= 488).

### Results

### E-iv) Myxothiazol production

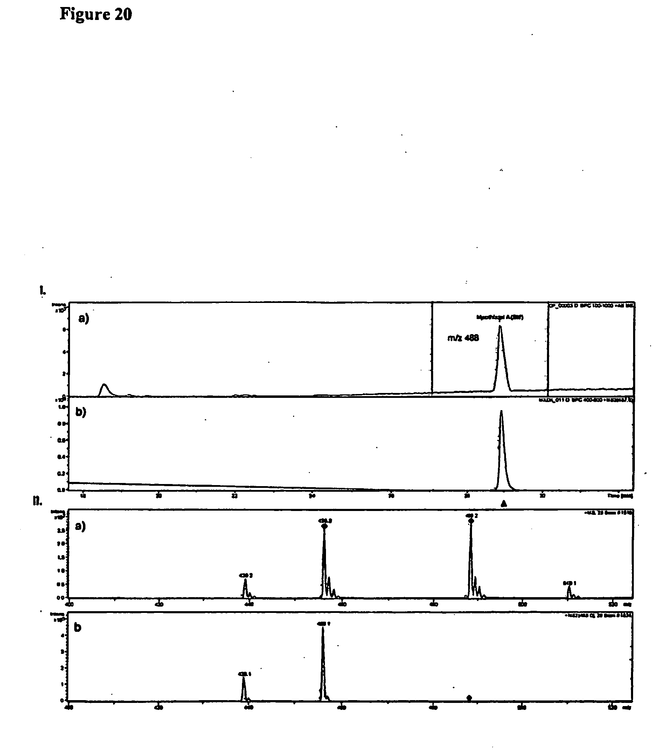

The introduction of the myxothiazol biosynthetic gene cluster into the chromosome of the *P*. *putida* FG2005 was verified genetically by colony PCR (data not shown). Positive clones were cultured in liquid media and the myxothiazol expression was induced with toluic acid. Followed HPLC-MS has shown in *P. putida* FG2005 the presence of myxothiazol, which could be detected by comparison with the reference standard (Figure 20). As the controls, *P. putida* wild-type strain, as well as the *P. putida* strain containing myxothiazol genes, but no exogenous genes involved in methylmalonate production, were used. As expected myxothiazol was not detected in extracts of either of these control strains, which are not able to synthesize methylmalonate and thus do not provide the substrate for biosynthesis of the compound.

### Example 5:

### Introduction of PKS/NRPS gene clusters into heterologous hosts by using the mariner transposable element

### A) Essential elements for DNA transfer.

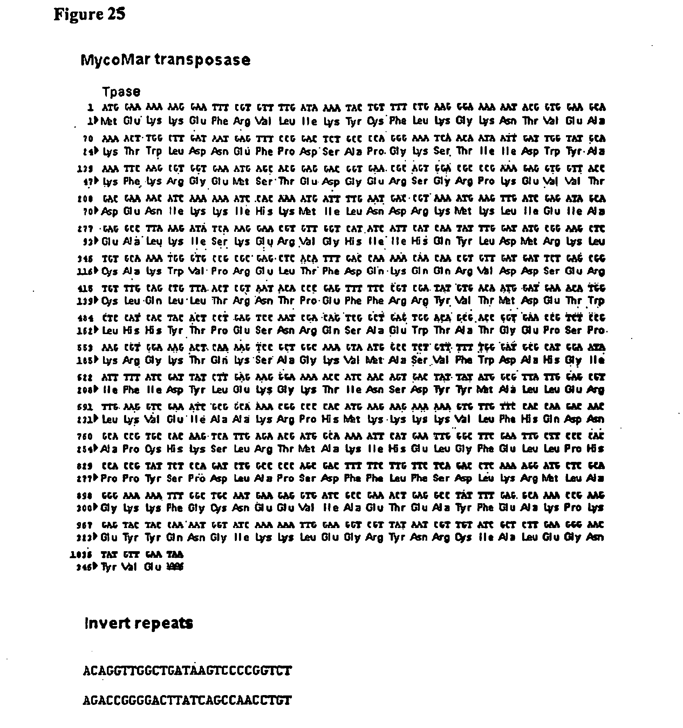

MycoMar transposase DNA and protein sequences and its inverted repeat sequence are shown in Figure 25.

### B) Engineering of myxochromide S (mchS) biosynthetic gene cluster

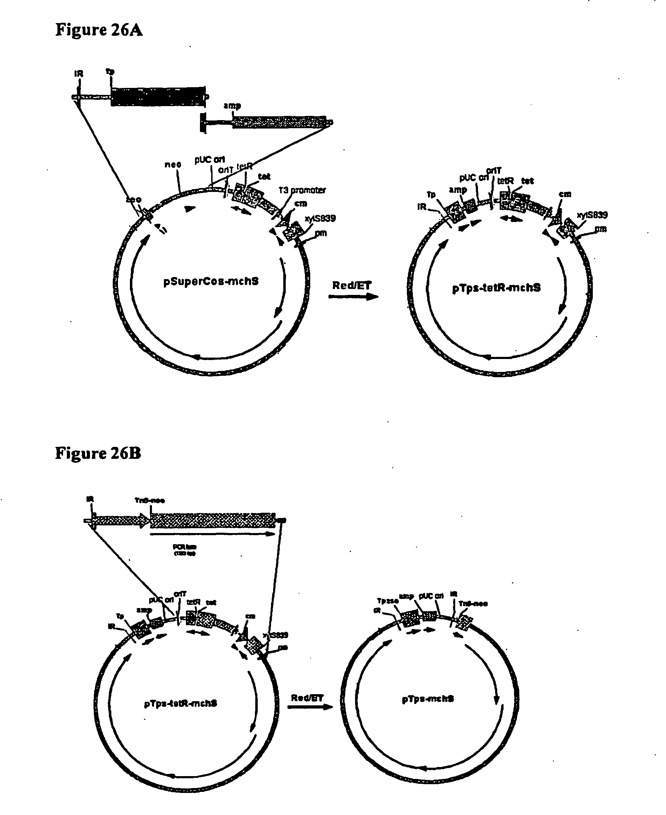

The myxochromide S *(mchS)* gene cluster is composed of 3 large genes and is 29.6kb in total. The starting construct comprising the *mchS* cluster is described in Wenzel, S. et al., "Heterologous expression of a myxobacterial natural products assembly line in Pseudomonads via Red/ET recombineering", Chemistry & Biology, 2005, 12: 349-356. Figure 26A shows the insertion of the MycoMar transposase gene into the *mchS* expression plasmid. The MycoMar transposase gene plus right *IR* fragment was generated by PCR reaction from an original MycoMar transposon vector (Rubin, E. et al., 1999, "In vivo transposition of mariner-based elements in enteric bacteria and mycobacteria", Proc. Natl. Acad. Sci. USA, 96:1645-1650). This PCR product, together with an *ampicillin* resistance gene PCR product, were inserted into the pSuperCos (Stratagene) backbone to delete the region containing the *zeocin* and *kanamycin* resistance genes using Red/ET recombination. This intermediate contains the left *IR* at the 3' end of the *mchS* gene cluster and the MycoMar transposase gene outside of the left *IR.*

The integration of the left IR plus *Tn5-neo* gene (which confers kanamycin resistance) in front of the *mchS* gene cluster is shown in Figure 26B. The previously-used cassette, *tetR-trpE-oriT* (Zhang Y et al., 2000, "DNA cloning by homologous recombination in Escherichia coli", Nature Biotechnology, 18:1314-1317), is not necessary for transposition and so was removed by the left *IR* plus *Tn5-neo* using Red/ET recombination. A ribosomal binding site was placed in front of the *mchS* gene cluster. The *Tn5* promoter will drive expression of the *neo* and *mchS* gene cluster (Figure 26B). The final construct is formed as *IR-Tn5-neo-mchS-IR-Tps* (transposase) and named pTps-mchS. The fragment inside of the two *IRs* will be integrated into the host chromosome by transposase.

### C) Integration of mchS gene cluster into Myxococcus xanthus genome;

*Myxococcus xanthus (M. xanthus)* can be transformed using electroporation. The construct, bearing homology arm(s), will be integrated into the chromosome via homologous recombination. However, as the efficiency of integration of large size DNA fragments into the chromosome is low, the correct clone, i.e., the clone containing the integrated large size DNA fragment, must be selected using a screening method. In contrast, transposition has been used frequently in *myxobacteria* for insertional mutagenesis and the transposition efficiency is much higher than the efficiency obtained using homologous recombination (Sandmann A et al., 2004, "Identification and analysis of the core biosynthetic machinery of tubulysin, a potent cytotoxin with potential anticancer activity" Chemistry and Biology. 11:1071-9; Kopp, M. et al., 2004, "Critical variations of conjugational DNA transfer into secondary metabolite multiproducing Sorangium cellulosum strains So ce12 and So ce56: development of a mariner-based transposon mutagenesis system", J Biotechnol., 107(1):29-40).

### C-i) Competent cells preparation and transformation

A small *M. xanthus* clump on a fresh plate was scraped into 1.4 ml medium in an eppendorftube with a punched hole on the lid. After 16 hours culturing at 32°C with 1,100 rpm shaking in a thermo-mixer (Eppendorf), the cells were pelleted at 10,000 rpm for 1 min in an Eppendorf centrifuge. The cell pellet was resuspended in cold dH₂O and spun down at 10,000 rpm for 1min. After washing twice with cold dH₂O, the cell pellet was suspended in 50 µl dH₂O. 3 µg of pTps-mchS plasmid DNA in 5 µl of 5mM Tris-HCI, pH8.0 buffer were added to the cells. The cells plus DNA were transferred into a pre-cold electroporation cuvette with 2 mm gap. All of the above steps were done on ice. Electroporation was carried out at 1,200 kv by using Eppendorf electroporator for bacterial cells. 1 ml of medium was added to the cuvette and the electroporated cells were transferred back into the eppendorf tube. 10 µl of culture were plated on kanamycin plate (50 µg/ml) with top agar after 5 hours culturing at 32°C with shaking in a thermomixer. Colonies were visible after 6 days incubation at 30°C.

### Results

To compare the homologous integration and transpositional integration, a homologous integration plasmid pOPB18 (6.7 kb), a small transposition plasmid pTps-lacZ (with 5.5 kb fragment inside of *IRs)* were used as control for transformation. The original pSuperCos-mchS (43 kb) plasmid which has neither homologous recombination nor transposition ability in *M. xanthus* was used as negative control. Table 2 shows the number of transformants obtained from transformation. The numbers are average transformants of 3 transformations carried out for each plasmid.

**Table 2**

| Plasmid: | pSupercos-mchS | pTps-mchS | pTps-lacZ | pOPB18 |
|---|---|---|---|---|
| Average number of transformants obtained from 3 transformations with plasmid: | 0 | 127 | 204 | 5 |

pTps-mchS is around 35 kb in total and the integration fragment inside of the two *IRs* is around 31kb. Although its efficiency of integration is lower than for the small integration fragment (pTps-lacZ), it is more efficient at integrating than the homologous integration plasmid (pOPB18).

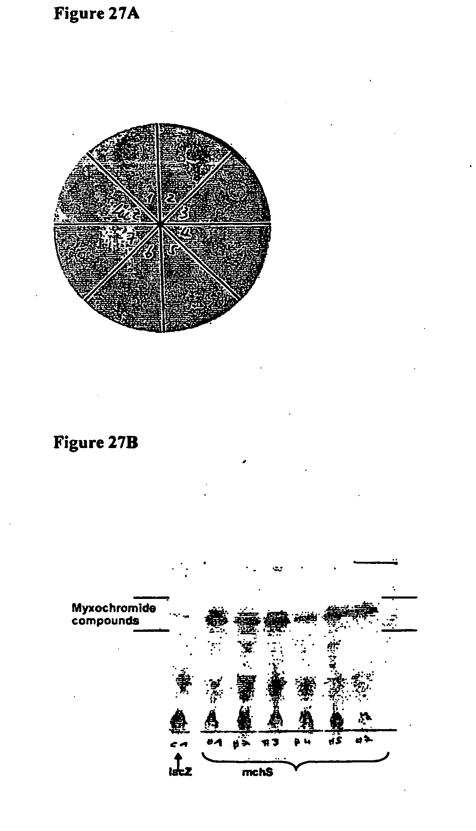

Myxochromide S compounds are characterized by their yellow-orange colour and are easy to observe in culture. Colonies from pTps-mchS and pTps-lacZ were picked and replated on kanamycin plate. The photo in Figure 27A was taken from the plate after incubation for 2 days. Clones 1-7 are from pTps-mchS transformation and /*acZ* is from pTps-lacZ transformation. Colonies from pTps-mchS transformation were truly redish (Figure 27A) and the liquid cultures were also redish (data not shown).

*MchS* and *lacZ* clones were cultured in 100 ml medium and the *m*y*xochromide* compounds were extracted from medium and cells. The compounds were run in a thin layer chromatography (TLC). The results are shown in Figure 27B. Lane 1 is *lacZ* clone as negative control. Lanes 2-7 are *mchS* clones 1-5 and 7, respectively. *Myxochromide* compounds are yellowish in TLC. *Myxochromide* compounds can be found in the cells and also in the growth medium. The secretion of the myxochromides into the culture medium may be useful for production of the compound as it may be captured using, for example, a resin, which might lead to loss of feedback inhibition.

### D) Detection of myxochromide S after introduction of the mchS pathway from S. aurantiaca into M.xanthus

A methanol extract from the *M. xanthus DK1622:mchS* mutant strain was analyzed with HPLC and HPLC/MS for the production or myxochromides S.

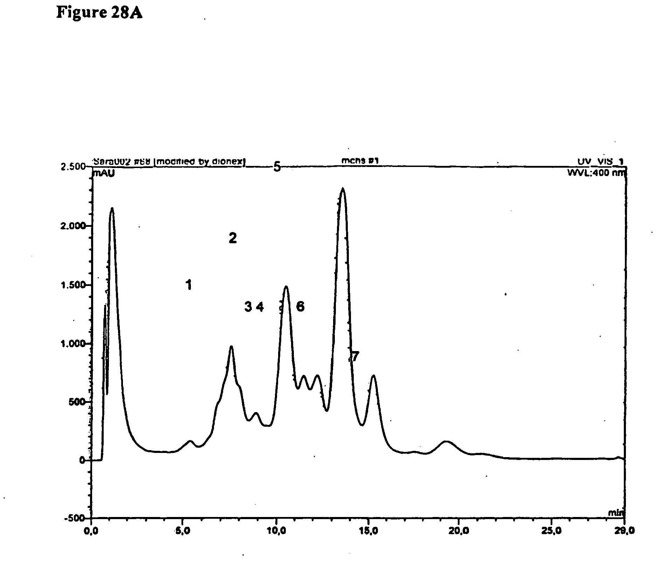

Using the HPLC conditions described below, myxochromides S₁₋₃, known from *S. aurantica,* could be identified in extracts of the *M. xanthus* mutant strains via HPLC (peaks 2 (S₁), 5 (S₂), 7 (S₃) shown in Figure 28A), which could also be verified via HPLC/MS analysis (data not shown).

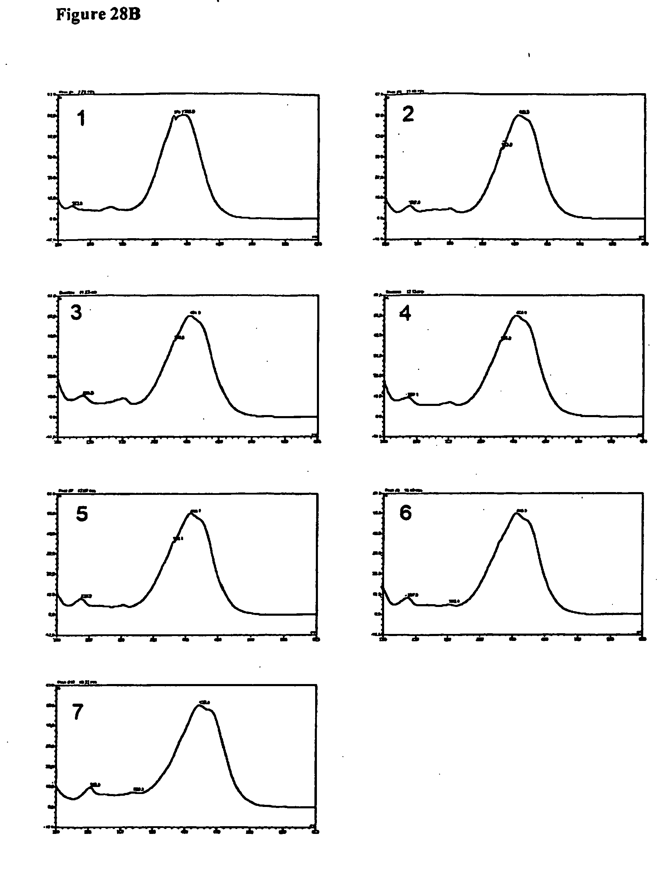

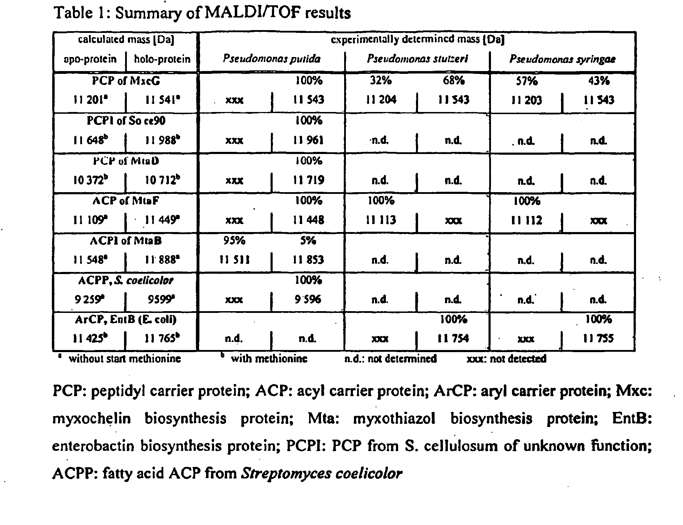

Due to the high production of myxochromides S in *M. xanthus* (> 500 mg/l), minor myxochromide S derivatives could also be detected (peaks 1, 3, 4 and 6 of Figure 28A). The uv spectra results shown in Figure 28B are typical uv spectra for myxochromides indicating that novel compounds with related structures are made.

### D-i) HPLC conditions:

HPLC was carried out using a DIONEX solvent system with a diode-array detector (PDA-100); column: MN nucleodur-C18 (RP) 125 x 2 mm /3 µm (precolumn: 8 x 3 mm / 5 µm); solvents: water + 0.1 % acetic acid (A) and acetonitril + 0.1 % acetic acid (B); solvent gradient from 50 % B at 2 min to 60 % B at 22 min and from 60 % B at 22 min to 95 % B at 26 min, followed by 3 min with 95 % B; flow rate: 0.4ml/min, detection at 400 nm.

### E) Transformation of pTps-mchS into Pseudomonads

pTps-mchS has no *oriT* for conjugation and it must be transformed into *Pseudomonas putida.* The preparation of *P. putida* competent cells was the same as described in Example 4 for *methylmalonyl-CoA* production (see E-i). 3 µg of pTps-mchS plasmid DNA were electroporated into *P. putida* competent cells. Transformed cells were plated on kanamycin plate. Colonies were formed after incubation for one day at 30°C.

There were more than 100 colonies per transformation and these clones produced *myxochromide* compounds (data not shown).

### F) Transformation of pTps-mchS into Myxobacteria GT2

Myxobacteria GT2 was also transformed by the pTps-mchS construct and found to produce the myxochromide compounds (data not shown).

## Claims

1. A method for the heterologous expression of a secondary metabolite produced by a multi-gene biosynthetic pathway, comprising:
i) generating in a first host cell, a single vector comprising all the component genes of the biosynthetic pathway, wherein the vector is constructed using principles of recombineering;
ii) transforming a second host cell with the vector, wherein the second host cell is a *Pseudomonas* or *Myxobacterium;*
iii) culturing the second host cell under conditions which are suitable for synthesis of the secondary metabolite; and
wherein the genes of the biosynthetic pathway are transcribed under the control of promoters that are found naturally in the second host-cell.

2. A method for the heterologous expression of a secondary metabolite produced by a multi-gene biosynthetic pathway, comprising:
i) generating In a first host cell, a single vector comprising all the component genes of the biosynthetic pathway, wherein the vector is constructed using principles of recombineering,
ii) transforming a second host cell with the vector, wherein the second host cell is a *Pseudomonas* or *Myxobacterium;*
iii) culturing the second host cell under conditions which are suitable for synthesis of the secondary metabolite; and
wherein one or more genes in the biosynthetic pathway is under the control of an inducible promoter.

3. A method according to claim 1 or claim 2, wherein the biosynthetic pathway is a polyketide pathway, a non-ribosomal peptide (NRP) or a fatty acid pathway.

4. A method according to claim 3, wherein the biosynthetic pathway is a type I polyketide pathway.

5. A method according to any one of claims 1 to 4, wherein the component genes of the biosynthetic pathway are encoded by a stretch of DNA of 40-100 kb in length.

6. A method according to any one of the preceding claims, wherein the secondary metabolite is not naturally produced in the second host cell.

7. A method according to any one of claims 1 and 3-6, wherein one or more of the genes of the biosynthetic pathway is under the control of an inducible promoter.

8. A method according to claim 7, wherein the inducible promoter is activated by a small molecule.

9. A method according to any one of the preceding claims, wherein the vector is a BAC.

10. A method according to any one of the preceding claims, wherein the component genes of the biosynthetic pathway are comprised within a transposable element carried by the vector.

11. A method according to any one of the preceding claims, wherein the transposable element is the MycoMar transposable element.

12. A method according to claim 10 or claim 11, wherein the vector further comprises a suitable transposase.

13. A method according to any one of the preceding claims, wherein the first host cell is *E*. *coli* or *Salmonella.*

14. A method according to any of the preceding claims, wherein the method is performed in iterative steps of screening and selection.

15. A method according to any preceding claims wherein the second host is transformed with genes encoding the enzymes required for making substrates that are required to synthesise the secondary metabolite but which are not endogenously expressed in the second host cell.

16. A method according to claim 15, wherein the second host cell is a Pseudomonas and the Pseudomonas is transformed with the genes encoding the enzymes required to synthesize methylmalonyl-CoA.

## Patentansprüche

1. Verfahren zur heterologen Expression eines sekundären Metaboliten, der durch einen multigenischen Biosyntheseweg hergestellt wird, umfassend:
i) Erzeugen eines einzelnen Vektors, der alle Gene umfaßt, die Bestandteil des Biosynthesewegs sind, in einer ersten Wirtszelle, wobei der Vektor unter Verwendung der Prinzipien des Recombineerings hergestellt wird,
ii) Transformieren einer zweiten Wirtszelle mit dem Vektor, wobei die zweite Wirtszelle ein *Pseudomonas* oder *Myxobacterium* ist,
iii) Kultivieren der zweiten Wirtszelle unter Bedingungen, die für die Synthese des sekundären Metaboliten geeignet sind, und
wobei die Gene des Biosynthesewegs unter der Kontrolle von Promotoren, die natürlich in der zweiten Wirtszelle gefunden werden, transkribiert werden.

2. Verfahren zur heterologen Expression eines sekundären Metaboliten, der durch einen multigenischen Biosyntheseweg hergestellt wird, umfassend:
i) Erzeugen eines einzelnen Vektors, der alle Gene umfaßt, die Bestandteil des Biosynthesewegs sind, in einer ersten Wirtszelle, wobei der Vektor unter Verwendung der Prinzipien des Recombineerings hergestellt wird,
ii) Transformieren einer zweiten Wirtszelle mit dem Vektor, wobei die zweite Wirtszelle ein *Pseudomonas* oder *Myxobacterium* ist,
iii) Kultivieren der zweiten Wirtszelle unter Bedingungen, die für die Synthese des sekundären Metaboliten geeignet sind, und
wobei ein oder mehrere Gen(e) in dem Biosyntheseweg unter der Kontrolle eines induzierbaren Promotors ist bzw. sind.

3. Verfahren nach Anspruch 1 oder Anspruch 2, wobei der Biosyntheseweg ein Polyketidweg, ein nichtribosomales Peptid- (NRP-) oder ein Fettsäureweg ist.

4. Verfahren nach Anspruch 3, wobei der Biosyntheseweg ein Typ I Polyketidweg ist.

5. Verfahren nach einem der Ansprüche 1 bis 4, wobei die Gene, die Bestandteile des Biosynthesewegs sind, durch einen DNA-Abschnitt von 40 bis 100 kb Länge kodiert werden.

6. Verfahren nach einem der vorstehenden Ansprüche, wobei der sekundäre Metabolit nicht natürlich in der zweiten Wirtszelle hergestellt wird.

7. Verfahren nach einem der Ansprüche 1 und 3 bis 6, wobei eines oder mehrere der Gene des Biosynthesewegs unter der Kontrolle eines induzierbaren Promotors ist.

8. Verfahren nach Anspruch 7, wobei der induzierbare Promotor von einem kleinen Molekül aktiviert wird.

9. Verfahren nach einem der vorstehenden Ansprüche, wobei der Vektor ein BAC ist.

10. Verfahren nach einem der vorstehenden Ansprüche, wobei die Gene, die Bestandteil des Biosynthesewegs sind, in einem transponierbaren Element, das von dem Vektor getragen wird, umfaßt sind.

11. Verfahren nach einem der vorstehenden Ansprüche, wobei das transponierbare Element das MycoMar transponierbare Element ist.

12. Verfahren nach Anspruch 10 oder Anspruch 11, wobei der Vektor weiter eine geeignete Transposase umfaßt.

13. Verfahren nach einem der vorstehenden Ansprüche, wobei die erste Wirtszelle *E. coli* oder *Salmonella* ist.

14. Verfahren nach einem der vorstehenden Ansprüche, wobei das Verfahren in sich wiederholenden Schritten des Screenings und der Selektion durchgeführt wird.

15. Verfahren nach einem der vorstehenden Ansprüche, wobei der zweite Wirt mit Genen transformiert wird, welche die Enzyme kodieren, die für das Herstellen von Substraten benötigt werden, die für die Synthese des sekundären Metaboliten benötigt werden, die aber nicht endogen in der zweiten Wirtszelle exprimiert werden.

16. Verfahren nach Anspruch 15, wobei die zweite Wirtszelle ein *Pseudomonas* ist und das *Pseudomonas* mit den Genen transformiert ist, die Enzyme kodieren, die für die Synthese von Methylmalonyl-CoA benötigt werden.

## Revendications

1. Procédé d'expression hétérologue d'un métabolite secondaire produit par une voie de biosynthèse multigénique, comprenant :
i) la génération, dans une première cellule hôte, d'un vecteur simple comprenant tous les gènes qui constituent la voie de biosynthèse, le vecteur étant construit en utilisant les principes de la recombinaison génétique ;
ii) la transformation d'une seconde cellule hôte avec le vecteur, la seconde cellule hôte étant issue de *Pseudomonas* ou de *Myxobacterium ;*
iii) la mise en culture de la seconde cellule hôte dans des conditions appropriées pour la synthèse du métabolite secondaire ; et
dans lequel les gènes de la voie de biosynthèse sont transcrits sous le contrôle de promoteurs naturellement présents dans la seconde cellule hôte.

2. Procédé d'expression hétérologue d'un métabolite secondaire produit par une voie de biosynthèse multigénique, comprenant :
i) la génération, dans une première cellule hôte, d'un vecteur simple comprenant tous les gènes qui constituent la voie de biosynthèse, le vecteur étant construit en utilisant les principes de la recombinaison génétique ;
ii) la transformation d'une seconde cellule hôte avec le vecteur, la seconde cellule hôte étant issue de *Pseudomonas* ou de *Myxobacterium ;*
iii) la mise en culture de la seconde cellule hôte dans des conditions appropriées pour la synthèse du métabolite secondaire ; et
dans lequel un ou plusieurs des gènes de la voie de biosynthèse est ou sont sous le contrôle d'un promoteur inductible.

3. Procédé selon la revendication 1 ou 2, dans lequel la voie de biosynthèse est une voie de biosynthèse des polykétides, des peptides non ribosomaux (NRP) ou des acides gras.

4. Procédé selon la revendication 3, dans lequel la voie de biosynthèse est une voie de biosynthèse des polykétides de type I.

5. Procédé selon l'une quelconque des revendications 1 à 4, dans lequel les gènes qui constituent la voie de biosynthèse sont codés par un segment d'ADN d'une longueur de 40 à 100 kb.

6. Procédé selon l'une quelconque des revendications précédentes, dans lequel le métabolite secondaire n'est pas naturellement produit dans la seconde cellule hôte.

7. Procédé selon l'une quelconque des revendications 1 et 3-6, dans lequel un ou plusieurs des gènes de la voie de biosynthèse est ou sont sous le contrôle d'un promoteur inductible.

8. Procédé selon la revendication 7, dans lequel le promoteur inductible est activé par une petite molécule.

9. Procédé selon l'une quelconque des revendications précédentes, dans lequel le vecteur est un vecteur BAC.

10. Procédé selon l'une quelconque des revendications précédentes, dans lequel les gènes qui constituent la voie de biosynthèse sont compris au sein d'un élément transposable porté par le vecteur.

11. Procédé selon l'une quelconque des revendications précédentes, dans lequel l'élément transposable est l'élément transposable MycoMar.

12. Procédé selon la revendication 10 ou 11, dans lequel le vecteur comprend en outre une transposase appropriée.

13. Procédé selon l'une quelconque des revendications précédentes, dans lequel la première cellule hôte est issue de *E*. *coli* ou de *Salmonella.*

14. Procédé selon l'une quelconque des revendications précédentes, lequel est réalisé par des étapes itératives de criblage et de sélection.

15. Procédé selon l'une quelconque des revendications précédentes, dans lequel la seconde cellule hôte est transformée par des gènes qui codent les enzymes requises pour la fabrication de substrats nécessaires à la synthèse du métabolite secondaire mais qui ne sont pas exprimés de façon endogène dans la seconde cellule hôte.

16. Procédé selon la revendication 15, dans lequel la seconde cellule hôte est issue de *Pseudomonas,* et dans lequel celle-ci est transformée par les gènes qui codent les enzymes nécessaires à la synthèse de la méthylmalonyl-CoA.
